Europäisches Patentamt

**(19)** European Patent Office

Office européen des brevets

**(11)** Publication number : **0 316 919 B1**

**(12)** # EUROPEAN PATENT SPECIFICATION

**(45)** Date of publication of patent specification :
07.06.95 Bulletin 95/23

**(51)** Int. Cl.⁶ : **G01N 33/76,** G01N 33/543

**(21)** Application number : **88119147.2**

**(22)** Date of filing : **17.11.88**

**(54) Vaginal sample test and reagents.**

**(30)** Priority : **17.11.87 US 121895**
**17.11.87 US 121900**
**17.11.87 US 121902**
**17.11.87 US 121893**
**17.11.87 US 121894**
**17.11.87 US 121899**
**15.09.88 US 244969**

**(43)** Date of publication of application :
**24.05.89 Bulletin 89/21**

**(45)** Publication of the grant of the patent :
**07.06.95 Bulletin 95/23**

**(84)** Designated Contracting States :
**AT BE CH DE ES FR GB GR IT LI LU NL SE**

**(56)** References cited :
**EP-A- 0 151 320**
**US-A- 3 171 783**

**(56)** References cited :
**PROC. NATL. ACAD. SCI. USA, vol. 82, October 1985, pages 6517-6521, Washington,US; H. MATSUURA et al.: "The oncofetal domain of fibronectin defined bymonoclonal antibody FDC-6: Its presence in fibronectins from fetal and tumortissues and its absence in those from normal adult tissues and plasma"**

**(73)** Proprietor : **ADEZA BIOMEDICAL CORPORATION**
**1240 Elko Street**
**Sunnyvale, California 94089 (US)**

**(72)** Inventor : **Teng, Nelson N.H.**
**24 Bluebell Lane**
**Hillsborough California 94010 (US)**
Inventor : **Senyei, Andrew E.**
**12962 Merriam Place**
**Santa Ana California 92705 (US)**

**(74)** Representative : **Stuart, Ian Alexander et al**
**MEWBURN ELLIS**
**York House**
**23 Kingsway**
**London WC2B 6HP (GB)**

## Description

This invention relates to methods, reagents and kits for detection of normal and ectopic pregnancy; the termination of pregnancy; and increased risk of preterm labor and rupture of membranes. Each embodiment of the invention involves procuring a test sample from the vaginal cavity to determine the presence or absence of a diagnostic indicator in the test sample.

In one embodiment which determines pregnancy during the first trimester of pregnancy, the test sample is assayed to determine the presence of a nonrestricted pregnancy indicator.

In an embodiment to determine pregnancy during the first 20 weeks of pregnancy, the presence of a fetal restricted antigen is determined.

A method for determining ectopic pregnancy comprises testing a test sample obtained from a pregnant patient during the first 20 weeks of pregnancy, to determine the absence of a fetal restricted antigen in the sample. The absence of fetal restricted antigen in the sample indicates the presence of an ectopic pregnancy.

This invention relates to detection of *ex vivo* products of conception. In particular, products of conception in uterine tissue expelled or removed from the uterus through a suspected spontaneous abortion (miscarriage) or a therapeutic abortion are determined.

An embodiment for detection of increased risk of preterm labor and rupture of the amniotic membrane after week 20 of pregnancy assays a test sample for the presence of a fetal restricted antigen.

A wide variety of tests have been developed for the determination of pregnancy. Commercial early pregnancy determinations generally involve assay of urine or serum. Home pregnancy tests for hCG (human chorionic gonadotropin) in urine include a variety of enzyme immunoassays, hemagglutination inhibition, and antibody-indicator agglutination tests which are effective to indicate pregnancy from 0 to 7 days after a missed period. Confirmation by physician is recommended, particularly to determine abnormal gestation such as ectopic pregnancy.

HCG is produced by the fetal trophoblast and passes from the fetal blood into the mother's blood through the intervillous space in the placenta. HCG levels in maternal blood and urine are often detectable at about 3 weeks. The sensitivity of serum or urine hCG tests is limited because the amount of hCG produced is determined by the amount of trophoblastic tissue, and by dilution of the hCG in the maternal fluids. Until development of the $\beta$-hCG specifically binding antibody, cross-reaction with LH (luteinizing hormone) also placed a limit on the level of sensitivity.

We have discovered that the status of normal uterine pregnancies can be determined early in the gestation cycle and with a high reliability by testing a sample removed from the vicinity of the cervical canal or cervical os for the presence of "pregnancy antigens", that is, antigenic and non-antigenic compounds or materials which are produced in the placental tissue and which are either not normally present in bodily fluids (plasma, serum or urine) when a woman is not pregnant, or which are present in maternal bodily fluids in increased amounts during pregnancy.

We have discovered that normal uterine pregnancies can be determined early in the gestation cycle and with a high reliability by testing a sample removed from the vicinity of the cervical canal or cervical os for the presence of fetal restricted antigens, that is, compounds or materials which are produced in the placental tissue and which do not pass in any substantial amounts into the maternal blood. Included in this class of antigens are fetal fibronectins.

We have discovered that ectopic pregnancy can be determined by testing a sample removed from the vicinity of the cervical canal or cervical os for the presence of fetal restricted antigens, that is, compounds or materials which are produced in the placental tissue and which do not pass in any substantial amounts into the maternal blood. Included in this class of materials are fetal fibronectins. If the fetal restricted antigens are substantially depressed in a sample from a person who is tested positive for pregnancy by a blood or urine test for nonrestricted pregnancy antigen, an ectopic pregnancy is indicated.

Determination of the presence of *ex vivo* products of conception in uterine tissue removed in therapeutic or spontaneous abortion is critically important to confirm the existence of uterine pregnancy and the termination thereof, and to rule out the presence of an ectopic pregnancy. If levels of fetal associated antigens in maternal serum or urine indicate pregnancy, and the uterine tissue removed during a therapeutic abortion contain no products of conception, a possible ectopic pregnancy is indicated. Presence of products of conception in uterine discharge associated with indicators of spontaneous abortion confirms the abortion, while the absence thereof indicates a continuation of pregnancy. Usual immunoassay techniques for determining the presence of fetal associated antigens in test samples derived from the vaginal cavity are not reliable for indicating the presence of products of conception since these samples typically contain maternal blood. Nonrestricted fetal antigens are usually present in maternal blood as well as in fetal and placental tissue.

Determination of impending preterm births is critical for increasing neonatal survival of preterm infants.

Detection of rupture of the amniotic membrane is important in distinguishing true and false labor. When the rupture is small and the volume of amniotic liquid escaping is small, the rupture is often undetected. Accepted methods for detecting ruptured membranes are subjective, not sufficiently sensitive, and not specific. An embodiment of this invention for detection of increased risk of preterm labor and rupture of the amniotic membrane after week 20 of pregnancy is directed to an assay of a test sample removed from the vicinity of the posterior fornix, cervical canal, or cervical os.

The method of this invention is used to determine the presence and/or status of a pregnancy. The method herein is used to determine the presence of a diagnostic indicator in a sample derived from the vaginal cavity, and comprises:

(a) a method for determining pregnancy during the first trimester comprising testing a test sample obtained from the vicinity of the cervical canal or cervical os, and determining the presence of a nonrestricted pregnancy antigen in the sample;

(b) a method for determining normal uterine pregnancy during the first 20 weeks of pregnancy comprising testing a test sample obtained from the vicinity of the cervical canal or cervical os, and determining the presence of a fetal restricted antigen in the sample;

(c) a method for determining fallopian pregnancy comprising testing a test sample obtained from the vicinity of the cervical canal or cervical os from a pregnant patient during the first 20 weeks of pregnancy, and determining the absence of a fetal restricted antigen in the sample;

(d) a method for determining ex vivo products of conception comprising testing a test sample expelled or removed from the uterus, and determining the presence of a fetal restricted antigen in the sample; or

(e) a method for determining increased risk of preterm labor or fetal membrane rupture comprising testing a test sample obtained from the vicinity of the posterior fornix, cervical canal or cervical os, from a patient after week 20 of pregnancy, and determining the presence of a fetal restricted antigen in the sample.

One embodiment of this invention for determining normal pregnancy during the first trimester of pregnancy comprises obtaining a test sample; and determining the presence of a nonrestricted pregnancy antigen in the sample. Examples of pregnancy antigens which are indicative of pregnancy when present in the test sample are hCG, hCT, hPL, SP1, PAPP-A, PAPP-B, HSAP, CAP, PP5, $PAMG_1$, $PAMG_2$, $\beta_1$-PAM, $\alpha_2$-PAM, hCLRF, somatostatin, MP1, PPI3, PP20, Protein B, and the like. In general, the embodiment of this invention which tests for a pregnancy antigen comprises interacting pregnancy antigen in a test sample with anti-(pregnancy antigen) antibody for a time sufficient to permit antigen-antibody binding to occur; and determining the presence or absence of said binding.

An embodiment for determining normal pregnancy during the first 20 weeks of pregnancy comprises obtaining a test sample; and determining the presence of a fetal restricted antigen in the sample. One fetal restricted antigen is fetal fibronectin. Since the fetal restricted antigen is not present in significant quantities in maternal plasma or serum, the methods of this embodiment are reliable even when the test sample is contaminated with maternal blood. In general, the embodiment which tests for normal pregnancy by assaying a fetal restricted antigen in a test sample comprises interacting the sample with an anti-(fetal restricted antigen) antibody for a time sufficient to permit antigen-antibody binding to occur; and determining the presence or absence of said binding.

The embodiment for detecting ectopic pregnancy during the first 20 weeks of pregnancy comprises testing a test sample obtained from a pregnant patient; and determining the absence of a fetal restricted antigen in the sample. In general, the embodiment which tests for ectopic pregnancy by assaying a fetal restricted antigen in a test sample comprises interacting the sample with an anti-(fetal restricted antigen) antibody for a time sufficient to permit antigen-antibody binding to occur; and determining the presence or absence of said binding.

The embodiment of this invention for determining the presence of products of conception in a test sample of tissue suspected to have been drawn from the uterus, i.e., derived from the uterus during a dilation and curettage (D&C), or therapeutic or suspected spontaneous abortion, comprises determining the presence in the sample of a fetal restricted antigen. In general, the embodiment which tests for ex vivo products of conception by assaying a fetal restricted antigen in a test sample comprises interacting the sample with an anti-(fetal restricted antigen) antibody for a time sufficient to permit antigen-antibody binding to occur; and determining the presence or absence of said binding.

The embodiment for determining fetal membrane rupture or an increased risk of preterm birth after week 20 of pregnancy comprises obtaining a test sample; and determining the presence in the sample of a fetal restricted antigen. In general, the embodiment which tests for increased risk of preterm labor and membrane rupture by assaying a fetal restricted antigen in a test sample comprises interacting the sample with an anti-(fetal restricted antigen) antibody for a time sufficient to permit antigen-antibody binding to occur; and determining the presence or absence of said binding.

Reagents for use with the assays of the invention include labeled and unlabeled anti-(pregnancy antigen)

3

antibodies such as anti-(hCG) antibodies, anti-(hPL) antibodies, and the like. Other reagents for use with the assays of the invention include insoluble supports to which are adhered the anti-(pregnancy antigen) antibodies such as anti-(hCG) antibodies, and the like. Labeled or unlabeled reagent pregnancy antigens are also reagents of this invention. Reagents for use with the assays of this invention also include insoluble supports to which are adhered reagent analyte, i.e., insoluble supports to which are adhered pregnancy antigen.

Reagents for use with the assays of the invention include labeled and unlabeled anti-(analyte) antibodies, i.e., anti-(fetal restricted antigen) antibodies such as anti-(fetal fibronectin) antibodies; anti-(analyte class) antibodies, i.e., anti-(fetal restricted antigen class) antibodies such as anti-(fibronectin) antibodies; and the like. Other reagents for use with the assays of the invention include insoluble supports to which are adhered the anti-(analyte) antibodies, i.e., anti-(fetal restricted antigen) antibodies such as anti-(fetal fibronectin) antibodies; anti-(analyte class) antibodies, i.e., anti-(fetal restricted antigen class) antibodies such as anti-(fibronectin) antibodies; and the like. Labeled or unlabeled reagent fetal restricted antigens are also reagents of this invention. Reagents for use with the assays of this invention also include insoluble supports to which are adhered reagent analyte, i.e., insoluble supports to which are adhered fetal restricted antigen.

This invention includes kits comprising one of the above reagents, alone or in combination with labeled antibodies. The reagents can be present in any suitable form in the kit, for example in containers, packages, and the like.

The method of this invention for determining the presence and/or status of a pregnancy comprises determining the presence of a diagnostic indicator, specifically a nonrestricted pregnancy antigen or a fetal restricted antigen, in a test sample removed from the vaginal cavity in the vicinity of the posterior fornix, cervical canal, or cervical os, and especially at the cervical canal or cervical os.

Specific embodiments of this invention are used to determine normal uterine pregnancy, ectopic pregnancy, the occurrence of a therapeutic or spontaneous abortion, and increased risk of preterm labor or rupture of the membranes.

One embodiment of this invention involves the detection of a pregnancy antigen in a test sample to determine pregnancy. We have discovered that detectable amounts of these materials are present in test samples removed in the vicinity of the cervical canal or cervical os during the first trimester of pregnancy, and are to be found in detectable levels in such samples early in gestation.

The term "pregnancy antigen" as used herein is defined to mean an antigenic or non-antigenic compound or substance which is formed either by placental tissue or by maternal tissue in the uterus in reaction to pregnancy, and which is present in the material sampled in accordance with this invention subsequent to such formation. Pregnancy antigens assayed to determine pregnancy are nonrestricted, that is, are present in maternal plasma, serum or urine in significant quantities. Prior to this invention, maternal serum, plasma or urine was tested for the presence of these materials to determine pregnancy. Since the quantity of the substances such as hCG produced are limited by the amount of placental tissue, the amounts produced in the first days following implantation are small and quickly diluted by maternal fluids. The pregnancy antigens appearing in the sample removed in the vicinity of the cervical canal or cervical os are not subject to gross dilution by maternal fluids, are apparently produced and released by the placental and maternal tissue in the uterine cavity, and are present in detectable amounts almost immediately after implantation or attachment of the morula (fertilized ovum) to the uterine endometrium. Included in the term "pregnancy antigen", as used herein, are antigenic materials, proteins, and other substances which are not antigenic in their purified form but which have unique epitopes to which antibodies can preferentially bind.

Examples of identified pregnancy antigens are human chorionic gonadotropin (hCG), human chorionic thyrotropin (hCT), human placental lactogen (hPL), Schwangerschafts-spezifizisches glykoprotein 1 or pregnancy specific $\beta_1$ glycoprotein (SP1), pregnancy-associated plasma protein A (PAPP-A), pregnancy-associated plasma protein B (PAPP-B), heat-stable alkaline phosphatase (HSAP) (S, I, and F phenotypes), cystine aminopeptidase (CAP), placental protein 5 (PP5), placenta specific $\alpha_1$-microglobulin (PAMG$_1$), placenta specific $\alpha_2$-microglobulin (PAMG$_2$), pregnancy associated $\beta_1$-macroglobulin ($\beta_1$-PAM), pregnancy associated $\alpha_2$-macroglobulin ($\alpha_2$-PAM), human chorionic luteinizing hormone-releasing factor (hCLRF), human chorionic thyrotropin-releasing hormone (hCTRH), human chorionic growth hormone-releasing inhibiting hormone (somatostatin), placental proteins PP20 and PP13, Protein B, and early pregnancy factor (EPF). Each of these are proteins which are produced by the placenta and which have been purified and well characterized.

The term "antibody" as used herein is defined to include antibodies of classes IgG, IgM, IgA, IgD, and IgE, and preferentially binding fragments and hybrid derivatives of antibodies, including Fab and F(ab')$_2$ fragments of antibodies. Antibodies may be polyclonal or monoclonal. Generally, monoclonal antibodies are preferred for use in the assays of this invention.

Immunological methods are most convenient for carrying out the assays of this invention because of their specificity, and the term "immunoassays" as used herein is defined to mean any method using a preferential binding of an antigen with a second material (i.e., a binding partner, usually an antibody or antibody fragment having an antigen binding site) which binds preferentially with an epitope of the antigen. Preferential binding as used herein refers to binding between binding partners which is selective and generally specific, and which demonstrates generally less than 10%, preferably less than 5%, cross-reactive nonspecific binding.

Included within the scope of this invention are all immunoassays including this step, including but not limited to sandwich, competition, dip stick, agglomeration, precipitation, transistor bridge probe, particle sorting, light disturbing, light scattering, and ultrasonic probe immunoassays, for example. Appropriate immunoassays may use, as labels, radioisotopes, enzymes, or fluorogenic, chromogenic, or chemiluminescent substances, for example.

A test sample which is to be assayed is removed in the vicinity of the cervical canal or cervical os, and the sample is assayed to determine the presence or quantity of pregnancy antigen in the sample. The sample generally comprises fluid and particulate solids, and may contain vaginal or cervical mucus, other vaginal or cervical secretions, cells or cell debris, amniotic fluid, or other fetal or maternal materials. The sample is removed with a swab having a dacron or other fibrous tip, aspirator, suction device, lavage device or the like and is transferred to a suitable container for storage and transport to the testing laboratory.

It is important that the test sample be dispersed in a liquid which preserves the sensitive protein analytes which are unstable in the sampled composition. The storage and transfer medium should prevent decline in the protein analyte level during storage and transport. A suitable preserving solution for storage and transfer consists of 0.05 M Tris-HCl, pH 7.4; 0.15 M NaCl, 0.02% $NaN_3$, 1% BSA, 500 Kallikrein Units/mL of aprotinin, 1 mM phenylmethylsulfonyl fluoride (PMSF) and 5 mM EDTA, and is described in U.S. Patent Application 244,969, filed September 15, 1988.

Detection of pregnancy antigens can be achieved by binding the pregnancy antigen in a test sample obtained in the vicinity of the cervical canal or cervical os, with an antibody which binds preferentially with an epitope of the pregnancy antigen, and determining the presence of this binding.

In a sandwich immunoassay embodiment of this embodiment, the test sample is contacted with an insoluble support to which anti-(pregnancy antigen) antibody is adhered to effect binding and capture of pregnancy antigen in the sample. The insoluble support is then contacted with an unlabeled or labeled antibody which binds with the pregnancy antigen adhering to the insoluble support to label and measure the captured pregnancy antigen, i.e., a secondary antibody. For example, anti-(hCG) antibody can be adhered to the insoluble support, and labeled or unlabeled anti-(hCG) antibody can be used to label the captured hCG antigen. The secondary antibody can have a physically detectable label which can be measured directly on the insoluble support. Alternatively, the secondary antibody can be unlabeled, and the secondary antibody can be determined by contacting the insoluble support with a labeled antibody or antibody fragment which binds selectively with the secondary antibody (i.e., a tertiary antibody), removing unbound labeled tertiary antibody from the support, and determining the presence of the label on the insoluble support. Sandwich immunoassays using a membrane substrate are appropriate for use.

Alternatively, the sample is tested by a competition immunoassay procedure. The sample can be mixed with labeled reagent antibody or antigen and incubated with an insoluble support to which an anti-(pregnancy antigen) antibody or reagent pregnancy antigen is adhered, competition occurring between the reagents for binding with the sample analyte. Methods and procedures to accomplish such immunoassays are well known to those skilled in the immunoassay art. The label ultimately adhering to the insoluble support or remaining in the solution is determined.

Anti-(hCG) polyclonal antibodies and their preparation are described in U.S. Patents 3,171,783, 3,234,096, 3,236,732, and 3,309,275. Anti-(hCG) monoclonal antibodies and their preparation are described in World Patent WO 8404598, Japanese Patent Application 62046262 (Feb. 28, 1987), and European Patent Application 210863 (Feb. 4, 1987). Anti-($\beta$-hCG) antibodies and their preparation are described in U.S. Patents 4,116,776, 4,123,509, 4,234,561, 4,256,629, 4,268,435, 4,310,455, and 4,313,871.

Other known pregnancy specific indicators and antibodies preferentially binding therewith are widely reported in the literature and patents. For example, anti-(SP1) antibodies and their preparation are described in Japanese Patent Applications 59174762 and 59214767, and in Engvall, E. et al, *Cancer Res.* (1982). Antibodies binding $\alpha$-fetoprotein and their preparation are described by Uotila, M. et al, *Mol.Immunol.* **17**:791 (1980) and Uotila, M. et al, *J.Immunol.Meth.* **42**:11 (1981). Anti-(EPF) (early pregnancy factor) antibodies and their preparation are described in World Patent WO 8605498. Anti-(hPL) (human placental lactogenic factor) antibodies are described, with their preparation, in U.S. Patent 3,892,841. Antibodies preferentially binding protein B are described in U.S. Patent 4,554,256. Anti-(MP1) (membrane associated placental protein) antibodies are described in European Patent Application 125514 (Nov. 21, 1984). Anti-(PP13) (placental specific protein) antibodies and their preparation are described in U.S. Patent 4,500,451. Anti-(PP17) antibodies and their preparation are described in U.S. Patent 4,468,345. Anti-(PP20) antibodies and their preparation are described in U.S. Patent 4,592,863. Anti-(PLAP) (placental alkaline phosphatase) antibodies and their preparation are described by Millan, J. et al, PLACENTAL PROTEINS (supra, referenced on p. 432). Antibodies which bind estrogen (estriol) and their preparation are described European Patent Application 178,683 (April 23, 1986). Antibodies which bind progesterone are described in Hungarian Patent Application T37028 (Nov. 28, 1985).

Anti-(pregnancy antigen) antibody can be obtained from pregnancy antigens, preferably from highly purified pregnancy antigens, by conventional antiserum or monoclonal techniques. This invention will be described herein with respect to the detection of human chorionic gonadotropin (hCG) as a pregnancy antigen, for purposes of clarity, and not by way of limitation: the detection of any pregnancy antigen is intended to be within the scope of this invention. The production of anti-(hCG) antibodies is described hereinabove.

Both monoclonal and polyclonal anti-(pregnancy antigen) antibodies can be derived directly from pregnancy antigens, preferably from highly purified antigens, by conventional antiserum or monoclonal techniques.

The principal antibodies useful in the assays of this invention are IgG and IgM antibodies, although the IgD, IgE and IgA antibodies can also be used if available in sufficient quantity. The antibodies are then affinity purified using conventional affinity chromatography techniques such as those described by Mishell and Shilgi in SELECTED METHODS IN CELLULAR IMMUNOLOGY. San Francisco: Freeman (1980), Goding, J., MONOCLONAL ANTIBODIES: PRINCIPLES AND PRACTICE. New York: Academic Press pp 111-114 (1983), and Parikh et al, *C&EN* (August 26, 1985).

Preferentially binding antibody fragments suitable for use in the kit and method of this invention can be made from the respective monoclonal or polyclonal antibodies by conventional enzyme or chemical fragmentation procedures. Suitable procedures are described by Tijssen, P. LABORATORY TECHNIQUES IN BIOCHEMISTRY AND MOLECULAR BIOLOGY: PRACTICE AND THEORIES OF ENZYME IMMUNOASSAYS. New York: Elsevier (1985), for example.

Polyclonal anti-(pregnancy antigen) antibody can be obtained by immunizing an animal such as a rabbit, guinea pig, rat or goat with concentrated pregnancy antigen, such as hCG, removing serum from the immunized animal, and separating the immunoglobulins from the serum, for example by ammonium sulfate precipitation.

Suitable absorbents for use in affinity chromatography include cross-linked agarose and cross-linked polyacrylamides to which the anti-(pregnancy antigen) antibody is covalently bonded. For removal of antibodies cross-reacting with other hormones or tissues, the antibody serum is passed through columns to which are coupled these materials. A portion of the eluant containing the remaining antibody can then be passed through a hCG column and eluted to yield the affinity purified antibody.

In these procedures, the antibody solution can be applied to the column in a phosphate buffered saline solution, and the antibodies can be eluted with a 2.5 M NaSCN solution, pH 8.0. Antibody concentration, if desired, can be achieved by negative pressure dialysis or ultrafiltration. The antibody solution is stable at temperature of 4°C or less. Repetition of the column separation procedures is continued until the desired separation and purity is achieved.

Monoclonal anti-(pregnancy antigen) antibody can be obtained by the methods of Galfre and Milstein, *Meth.Enzym.* **73**:1 (1981), immunizing mice with pregnancy antigens to obtain the spleen cells for hybridization. Suitable procedures are described by Goding, J. MONOCLONAL ANTIBODIES: PRINCIPLES AND PRACTICE. New York: Academic Press (1983) pp 56-97).

Sandwich Immunoassays: In a sandwich immunoassay embodiment of this invention to determine pregnancy antigen in a test sample, an insoluble support to which anti-(pregnancy antigen) antibody is adhered is contacted with a test sample diluted with an aqueous buffer solution such as phosphate buffer solution (PBS), pH 6 to 8 and preferably from 7.2 to 7.6, for a sufficient time to permit binding of pregnancy antigen in the test sample with the anti-(pregnancy antigen) antibody on the insoluble support. The sample is then removed from the support. The incubation time should be sufficient to permit substantial binding to occur, the time being temperature dependent. Suitable incubation times are from 30 to 240 minutes at temperatures within the range of from 16 to 40°C, the preferred contact time being at least 60 minutes at temperatures within the range of from 20 to 26°C.

The residual sample solution is then removed from the support by use of a rinse solution. Any conventional rinse solution can be used. A suitable rinse solution is described in U.S. Patent 4,528,267. It is an aqueous phosphate buffer solution having a phosphate molarity of from 0.0001 to 0.05, a pH of from 6 to 8 and containing from 0.001 to 0.1 weight percent of nonionic surfactant. Suitable nonionic surfactants include polyoxyethylene ethers (BRIJ Trade Marks such as lauryl, cetyl, oleyl, stearyl, and tridecyl polyoxyethylene ethers); polyoxyethylene sorbitans (TWEEN Trade Marks such as polyoxyethylene sorbital monolaurate, monopalmitate, monostearate, monoleate, and trioleates); and other polyoxyethylene ethers (TRITON Trade Marks, for example). A preferred nonionic surfactant is octylphenoxypolyethoxy ethanol having 40 ethylene oxide units (TRITON X-405 Trade Marks, Rohm and Hass Company).

The-insoluble support is then contacted with an antibody which will bind with the captured pregnancy antigen on the insoluble support, i.e., the sandwiching antibody. The sandwiching antibody can be labeled or unlabeled. In the event that an unlabeled sandwiching antibody is used, a tertiary antibody which binds with the sandwiching antibody and which bears a physically detectable label can be used in a conventional manner to determine the sandwiching antibody.

A variety of labels are described herein. For purposes of clarity and not by way of limitation, the subsequent steps of the process will be described for anti-(pregnancy antigen) antibodies which have been labeled with an enzyme, preferably a chromogenic or a fluorogenic enzyme. The term "chromogenic enzyme" is defined herein to refer to an enzyme which will produce a chromophore product with a suitable substrate. The term "fluorogenic enzyme" is defined herein to refer to an enzyme which will produce a fluorophore product with a suitable substrate.

The sandwiching antibody is applied to the insoluble support in an aqueous solution. The solution preferably contains suitable salts and buffers to preserve the reactants and facilitate the binding reaction. For example, the solution can contain bovine serum albumin (BSA), phosphate buffer solution (PBS), and a mild surfactant such as polyoxyethylene sorbitan ester employed in the above-described rinse solution. The incubation is continued for sufficient time to permit the sandwiching antibody to bind with exposed pregnancy antigen epitopes, if any, adhering to the insoluble support. The preferred incubation times and temperatures are as set forth for the binding of insolubilized reagent anti-(pregnancy antigen) antibody with the pregnancy antigen. The sandwiching antibody solution is then removed from the insoluble support, and the support is rinsed with a rinse solution such as described above, to remove any residual, unbound material.

If the sandwiching antibody is unlabeled, an enzyme labeled antibody or other binding agent which binds selectively with the sandwiching antibody is applied to the insoluble support in an aqueous solution. The solution preferably contains suitable salts and buffers to preserve the reactants and facilitate the binding reaction, such as described above. The incubation is continued for sufficient time to permit labeled anti-(sandwiching antibody) antibody to bind with exposed sandwiching antibody epitopes, if any, adhering to the insoluble support. The preferred incubation times and temperatures are as set forth for the binding of insolubilized reagent anti-(pregnancy antigen) antibody with the sample pregnancy antigen. The labeled antibody solution is then removed from the insoluble support, and the support is rinsed with a rinse solution such as described above, to remove any residual, unbound labeled material.

In a next step of the sandwich process, the insoluble support is contacted with an aqueous solution of a substrate which undergoes a reaction in the presence of the enzyme to release a fluorescent or chromogen compound into the solution. Suitable substrates and the enzymes which which they can be converted are described in U.S. Patents 4,190,496 and 4,528,267, for example. The support is contacted with an aqueous solution of the substrate containing from $10^{-2}$ to $10^{-10}$ molar concentrations of the substrate. Substrate molar concentrations of from $10^{-4}$ to $10^{-5}$ are preferred. Preferred additional reagents and buffers in the substrate solution include 2-amino-2-methyl-1-propanol buffer, TRIS, and magnesium chloride, for example.

The substrate solution is incubated with the insoluble support for sufficient time for the reaction yielding the fluorophore or chromophore to occur. At temperatures of from 18 to 40°C, incubation times of from 5 to 240 minutes can be used. Preferably, the temperature is within the range of from 20 to 26°C, and the incubation time is from 30 to 90 minutes.

The fluorescent or chromophore level in the solution is then measured. The equipment and procedures for determining the level of fluorescence or chromophore level in the substrate solutions are those conventionally used in the art. The level of fluorescence or chromogen in solution is a function of the enzyme concentration on the insoluble support which is, in turn, a function of the amount of pregnancy antigen in the test sample. The concentration of the pregnancy antigen can be determined by comparing the fluorescence or chromophore level of the solution with respective fluorescence or chromophore levels obtained with control solutions containing known concentrations of pregnancy antigen.

Membrane Immunoassays: In a membrane embodiment of this invention to determine pregnancy antigen in a test sample, an insoluble support to which anti-(pregnancy antigen) antibody is adhered is contacted with a test sample diluted with an aqueous buffer solution such as phosphate buffer solution (PBS), pH 6 to 8 and preferably from 7.2 to 7.6, for a sufficient time to permit binding of pregnancy antigen in the sample with the anti-(pregnancy antigen) antibody on the insoluble support. The time required for binding is very small in a flow through system. Suitable incubation times can be one second up to 20 minutes at temperatures within the range of from 16 to 40°C, the preferred contact time being less than one minute and optimally from 10 seconds to 2 minutes.

The insoluble support is then contacted with an antibody which will bind with the captured pregnancy antigen on the insoluble support, i.e., the sandwiching antibody. The sandwiching antibody can be labeled or unlabeled. In the event that an unlabeled sandwiching antibody is used, a tertiary antibody which binds with the sandwiching antibody and which bears a physically detectable label can be used in a conventional manner to determine the sandwiching antibody.

A variety of labels are described herein. For purposes of clarity and not by way of limitation, the subsequent steps of the process will be described for anti-(pregnancy antigen) antibodies which have been labeled with an enzyme, preferably a fluorogenic or chromogenic enzyme.

The sandwiching antibody is applied to the insoluble support in an aqueous solution. The solution preferably contains suitable salts and buffers to preserve the reactants and facilitate the binding reaction. For example, the solution can contain bovine serum albumin (BSA), phosphate buffer solution (PBS), and a mild surfactant such as polyoxyethylene sorbitan ester employed in the above-described rinse solution. The incubation is continued for sufficient time to permit the sandwiching antibody to bind with exposed pregnancy antigen epitopes, if any, adhering to the insoluble support. The preferred incubation times and temperatures are as set forth for the binding of insolubilized reagent anti-(pregnancy antigen) antibody with the test sample pregnancy antigen.

The sandwiching antibody solution optionally can be removed from the insoluble support, and the support is rinsed with a rinse solution such as described above, to remove any residual, unbound labeled material.

If the sandwiching antibody is unlabeled, an enzyme labeled antibody or other binding agent which binds selectively with the sandwiching antibody is applied to the insoluble support in an aqueous solution. The solution preferably contains suitable salts and buffers to preserve the reactants and facilitate the binding reaction, such as described above. The incubation is continued for sufficient time to permit labeled anti-(sandwiching antibody) antibody to bind with sandwiching antibody epitopes, if any, adhering to the insoluble support. The preferred incubation times and temperatures are as set forth for the binding of insolubilized reagent anti-(pregnancy antigen) antibody with the test sample pregnancy antigen.

The labeled antibody solution is then removed from the insoluble support, and the support is rinsed with a rinse solution such as described above, to remove any residual, unbound labeled material.

In a next step of the membrane sandwich process of this invention, the insoluble support is contacted with an aqueous solution of a substrate which undergoes a reaction in the presence of the enzyme to release fluorogen or chromogen compound into the solution. Suitable substrates and the enzymes which which they can be converted, as well as additional components and buffers have been described above.

The substrate solution is incubated with the insoluble support for sufficient time for the reaction yielding the fluorophore or chromophore to occur. At temperatures of from 18 to 40°C, incubation times of from 1 to 20 minutes can be used. Preferably, the temperature is within the range of from 20 to 26°C, and the incubation time is from 2 to 5 minutes. The fluorogen or chromogen level on the membrane can be measured using a reflectometer or densitometer.

Competition Immunoassays: Competition embodiments of this invention using labeled reagent pregnancy antigen comprise contacting a mixture of the test sample and the labeled reagent pregnancy antigen with an anti-(pregnancy antigen) antibody adhered to an insoluble support, and determining the amount of label which either binds with the insoluble support or remains in the solution phase.

The competition embodiments of this invention using labeled anti-(pregnancy antigen) antibodies can be of more than one form. One embodiment using anti-(pregnancy antigen) antibody bound to the insoluble support comprises contacting a mixture of the test sample and labeled anti-(pregnancy antigen) antibodies with an anti-(pregnancy antigen) antibody adhered to an insoluble support, and determining the amount of label which either binds with the insoluble support or remains in the solution phase. Another embodiment using reagent pregnancy antigen bound to the insoluble support comprises contacting a mixture of the test sample and labeled anti-(pregnancy antigen) antibodies with a pregnancy antigen adhered to an insoluble support, and determining the amount of label which either binds with the insoluble support or remains in the solution phase.

In each of these methods, the test sample is diluted with buffer solution, incubated and the label determined as described above with respect to the sandwich immunoassay embodiments. The concentration of the limiting reagent is selected to permit competitive binding between the reagents, with the amount of label remaining on the insoluble support or in the solution being a variable which is a function of the amount of the analyte in the sample. These methods are generally well known and how to vary them to optimize a procedure are fully within the knowledge of a person skilled in the immunoassay art.

The binding of the anti-(pregnancy antigen) antibody and the pregnancy antigen in the test sample can also be determined by agglomeration of particles to which the anti-(pregnancy antigen) antibody is adhered by pregnancy antigen in the sample, precipitation of antibodies due to antibody-antigen reactions, or observations of physical or electrical changes which occur upon the antibody-antigen binding, using semiconductor bridge probes, light disturbing patterns such as are described in U.S. Patent 4,647,544, and the like.

A determination of the presence of nonrestricted pregnancy antigen in the test sample indicates pregnancy.

Nonrestricted Pregnancy Antigen Test Kits included within the scope of this invention include combinations of a sampling device with (a) an anti-(pregnancy antigen) antibody adhered to an insoluble support; (b) a combination of anti-(pregnancy antigen) antibody adhered to an insoluble support, and either a labeled anti-(pregnancy antigen) antibody or a labeled reagent pregnancy antigen; or (c) a reagent pregnancy antigen adhered to an insoluble support and a labeled anti-(pregnancy antigen) antibody.

The kits of this invention can further comprise combinations to determine both of a pregnancy antigen and a fetal restricted antigen in a test sample; buffers for sample transport and storage; vials, foil packages or other containers of reagents of this invention; other, optional reagents such as enzyme substrate reagents in separate vials or other packages; mechanical or optical devices to determine the presence and extent of antibody-antigen binding; and combinations thereof. Sampling devices such as sampling swabs, and buffers for transport and storage can also be included. The individual parts of the kit may be packaged in any convenient form, such as vials, foil packages, or other containers. For example, insoluble support structures in a foil package can be combined with other reagents in vials or other packages.

A pregnancy testing embodiment involves the detection of a fetal restricted antigen, that is, a uniquely fetal or placental focused material, in a test sample removed in the vicinity of the cervical canal or cervical os. We have discovered that detectable amounts of these materials are present in such a sample during the first 20 weeks of pregnancy. Since the fetal restricted antigens are not present in significant quantities in the maternal blood, the presence of maternal blood in the sample does not interfere with the test.

The term "fetal restricted antigen" as used herein is defined to mean a uniquely fetal or placental derived material, which is either not present in maternal serum, plasma or urine, or is not present in significant amounts in maternal serum, plasma or urine. Any substance meeting this definition is intended to be included within the meaning of the term, including both antigenic materials and proteins and other substances which are not antigenic in their purified form but which have unique epitopes which can be selectively bound with antibodies specific or selective thereto. An example of a fetal restricted antigen is the fetal fibronectin which binds specifically with the FDC-6 monoclonal antibody described by H. Matsuura and S. Hakomori, *Proc.Natl.Acad.Sci.USA* **82**:6517-6521 (1985).

The term "fetal restricted antigen class" as used herein is defined to mean a class or group of antigens of which the fetal restricted antigen is a member. For example, fetal fibronectin is a fetal restricted member of the human fibronectin group or class.

The term "antibody" as used herein is defined to include antibodies of classes IgG, IgM, IgA, IgD, and IgE, and preferentially binding fragments and hybrid derivatives of antibodies, including Fab and $F(ab')_2$ fragments of antibodies. Antibodies may be polyclonal or monoclonal. Generally, monoclonal antibodies are preferred for use in the assays of this invention.

Immunological methods are most convenient for carrying out the assays of this invention because of their specificity, and the term "immunoassays" as used herein is defined to mean any method using a preferential binding of an antigen with a second material (i.e., a binding partner, usually an antibody or antibody fragment having an antigen binding site) which binds preferentially with an epitope of the antigen. Preferential binding as used herein refers to binding between binding partners which is selective and generally specific, and which demonstrates generally less than 10%, preferably less than 5%, cross-reactive nonspecific binding. For example, when the analyte is fetal fibronectin, the anti-(fetal fibronectin) antibody is less than 10%, and preferably less than 5%, cross-reactive with adult fibronectins.

Included within the scope of this invention are all immunoassays including this step, including but not limited to sandwich, competition, dip stick, agglomeration, precipitation, transistor bridge probe, particle sorting, light disturbing, light scattering, and ultrasonic probe immunoassays, for example. Appropriate immunoassays may use, as labels, radioisotopes, enzymes, or fluorogenic, chromogenic, or chemiluminescent substances, for example.

A test sample which is to be assayed is removed in the vicinity of the cervical canal or cervical os, and the sample is assayed to determine the presence or quantity of fetal restricted antigen in the sample. The sample generally comprises fluid and particulate solids, and may contain vaginal or cervical mucus, other vaginal or cervical secretions, cells or cell debris, amniotic fluid, or other fetal or maternal materials. The sample is removed with a swab having a dacron or other fibrous tip, aspirator, suction device, lavage device or the like and is transferred to a suitable container for storage and transport to the testing laboratory.

It is important that the test sample be dispersed in a liquid which preserves the sensitive protein analytes which are unstable in the sampled composition. The storage and transfer medium should prevent decline in the protein analyte level during storage and transport. A suitable preserving solution for storage and transfer consists of 0.05 M Tris-HCl, pH 7.4; 0.15 M NaCl, 0.02% $NaN_3$, 1% BSA, 500 Kallikrein Units/mL of aprotinin, 1 mM phenylmethylsulfonyl fluoride (PMSF) and 5 mM EDTA, and is described in U.S. Patent Application 244,969, filed September 15, 1988.

Detection of fetal restricted antigen can be achieved by binding the fetal restricted antigen in a test sample with an antibody which binds preferentially with an epitope of the fetal restricted antigen, and determining the presence or absence of this binding.

In one sandwich assay for fetal restricted antigen, the test sample is contacted with an insoluble support to which anti-(fetal restricted antigen) antibody is adhered to effect binding of fetal restricted antigen in the sample to the insoluble support. The insoluble support is then contacted with a secondary antibody, an unlabeled or labeled anti-(fetal restricted antigen) antibody, which binds with the fetal restricted antigen adhering to the insoluble support to label and measure the captured fetal restricted antigen.

An antibody which binds with a class of substances including the analyte fetal restricted antigen can be substituted for the specific anti-(fetal restricted antigen) antibody capture antibody or the specific anti-(fetal restricted antigen) antibody sandwiching antibody. For example, anti-(fetal fibronectin) antibody can be adhered to the insoluble support, and labeled or unlabeled anti-(fibronectin) antibody can be used to label the captured antigen. Alternatively, anti-(fibronectin) antibody can be adhered to the insoluble support, and labeled or unlabeled anti-(fetal fibronectin) antibody is used to label the captured antigen. The secondary antibody can have a physically detectable label which can be measured directly on the insoluble support. Alternatively, the secondary antibody can be unlabeled, and the secondary antibody can be determined by contacting the insoluble support with a labeled antibody or antibody fragment which binds selectively with the secondary antibody (i.e., a tertiary antibody), removing unbound labeled tertiary antibody from the support, and determining the presence of the label on the insoluble support. Sandwich immunoassays using a membrane substrate are appropriate for use.

The sample can also be tested by a competition immunoassay procedure. The test sample is mixed with labeled reagent antibody or antigen and incubated with an insoluble support to which an anti-(fetal restricted antigen) antibody or reagent fetal restricted antigen is adhered, competition occurring between the reagents for binding with the sample analyte. Methods and procedures to accomplish such immunoassays are well known to those skilled in the immunoassay art. The label ultimately adhering to the insoluble support or remaining in the solution is determined.

Anti-(fetal restricted antigen) antibody can be obtained from fetal restricted antigens, preferably from highly purified fetal restricted antigens, by conventional antiserum or monoclonal techniques. This invention will be described herein with respect to the detection of fetal fibronectin as a fetal restricted antigen, for purposes of clarity, and not by way of limitation: the detection of any fetal restricted antigen is intended to be within the scope of this invention. Fetal fibronectin is purified from amniotic fluid as described by Engvall and Ruoslahti, *Int.J.Cancer.* **20**:1-5 (1977). Anti-(fetal fibronectin) antibody can be derived from fetal fibronectin by conventional antiserum techniques or by monoclonal antibody techniques.

Both monoclonal and polyclonal anti-(fetal restricted antigen) antibodies, or anti-(fetal restricted antigen class) antibodies can be derived directly from fetal restricted antigens, preferably from highly purified antigens, by conventional antiserum or monoclonal techniques.

The principal antibodies useful in the assays of this invention are IgG and IgM antibodies, although the IgD, IgE and IgA antibodies can also be used if available in sufficient quantity. The antibodies are then affinity purified using conventional affinity chromatography techniques such as those described by Mishell and Shilgi in SELECTED METHODS IN CELLULAR IMMUNOLOGY. San Francisco: Freeman (1980), Goding, J., MONOCLONAL ANTIBODIES: PRINCIPLES AND PRACTICE. New York: Academic Press pp 111-114 (1983), and Parikh et al, **C&EN** (August 26, 1985).

Preferentially binding antibody fragments suitable for use in the kit and method of this invention can be made from the respective monoclonal or polyclonal antibodies by conventional enzyme or chemical fragmentation procedures. Suitable procedures are described by Tijssen, P. LABORATORY TECHNIQUES IN BIOCHEMISTRY AND MOLECULAR BIOLOGY: PRACTICE AND THEORIES OF ENZYME IMMUNOASSAYS. New York: Elsevier (1985), for example.

Polyclonal anti-(fetal restricted antigen) antibody can be obtained by immunizing an animal such as a rabbit, guinea pig, rat or goat with concentrated fetal restricted antigen, such as fetal fibronectin, removing serum from the immunized animal, and separating the immunoglobulins from the serum, for example by ammonium sulfate precipitation.

Suitable absorbents for use in affinity chromatography include cross-linked agarose and cross-linked polyacrylamides to which the fetal restricted antigen antibody is covalently bonded. For removal of antibodies cross-reacting with adult fibronectins, the antibody serum is passed through columns to which are coupled adult fibronectins. A portion of the eluant containing the remaining antibody can then be passed through a fetal fibronectin column and eluted to yield the affinity purified antibody.

In these procedures, the antibody solution can be applied to the column in a phosphate buffered saline solution, and the antibodies can be eluted with a 2.5 M NaSCN solution, pH 8.0. Antibody concentration, if desired, can be achieved by negative pressure dialysis or ultrafiltration. The antibody solution is stable at temperature of 4°C or less. Repetition of the column separation procedures is continued until the desired separation and purity is achieved.

For production of fetal fibronectin, the procedures described by H. Matsuura and S. Hakomori, *Proc.Natl.Acad.Sci.USA.* **82**:6517-6521 (1985) can be followed, replacing the tumor fibronectin with fetal fibronectin. Anti-(fetal restricted antigen class) antibodies of both polyclonal and monoclonal varieties are generally well known and available either commercially or from publicly available hybridoma deposits. For example, anti-(fibronectin) monoclonal antibodies can be derived from clone samples from ATCC HB 91 (American Type Culture Collection, Rockville, MD). Other such antibodies are described in Japanese Patent Application 60091264 (DIALOG database file 351, WPI Acc. No. 85-161617/27) and U.S. Patent 4,325,867.

Sandwich Immunoassays: In a sandwich embodiment of this invention to determine fetal restricted antigen in a test sample, an insoluble support to which anti-(fetal restricted antigen) antibody is adhered is contacted with a test sample diluted with an aqueous buffer solution such as phosphate buffer solution (PBS), pH 6 to 8 and preferably from 7.2 to 7.6, for a sufficient time to permit binding of fetal restricted antigen in the test sample with the anti-(fetal restricted antigen) antibody on the insoluble support, and then removing the sample from the support. The incubation time should be sufficient to permit substantial binding to occur, the time being temperature dependent. Suitable incubation times are from 30 to 240 minutes at temperatures within the range of from 16 to 40°C, the preferred contact time being at least 60 minutes at temperatures within the range of from 20 to 26°C.

Residual sample solution is then removed from the support by use of a rinse solution. Any conventional rinse solution can be used. A suitable rinse solution is described in U.S. Patent 4,528,267. It is an aqueous phosphate buffer solution having a phosphate molarity of from 0.0001 to 0.05, a pH of from 6 to 8 and containing from 0.001 to 0.1 weight percent of nonionic surfactant. Suitable nonionic surfactants include polyoxyethylene ethers (BRIJ Trade Marks such as lauryl, cetyl, oleyl, stearyl, and tridecyl polyoxyethylene ethers; polyoxyethylene sorbitans (TWEEN Trade Marks such as polyoxyethylene sorbital monolaurate, monopalmitate, monostearate, monoleate, and trioleates); and other polyoxyethylene ethers (TRITON Trade Marks, for example). A preferred nonionic surfactant is octylphenoxypolyethoxy ethanol having 40 ethylene oxide units (TRITON X-405 Trade Marks, Rohm and Hass Company).

The insoluble support is then contacted with an antibody which will bind with the captured fetal restricted antigen on the insoluble support, i.e., the sandwiching antibody. The sandwiching antibody can be an anti-(fetal restricted antigen) antibody, or can be an anti-(fetal restricted antigen class) antibody. The sandwiching antibody can be labeled or unlabeled. In the event that an unlabeled sandwiching antibody is used, a tertiary antibody which binds with the sandwiching antibody and which bears a physically detectable label can be used in a conventional manner to determine the sandwiching antibody.

A variety of labels are described herein. For purposes of clarity and not by way of limitation, the subsequent steps of the process will be described for anti-(fetal restricted antigen) antibodies which have been labeled with an enzyme, preferably a chromogenic or a fluorogenic enzyme. The term "chromogenic enzyme" is defined herein to refer to an enzyme which will produce a chromophore product with a suitable substrate. The term "fluorogenic enzyme" is defined herein to refer to an enzyme which will produce a fluorophore product with a suitable substrate.

The sandwiching antibody is applied to the insoluble support in an aqueous solution. The solution preferably contains suitable salts and buffers to preserve the reactants and facilitate the binding reaction. For example, the solution can contain bovine serum albumin (BSA), phosphate buffer solution (PBS), and a mild surfactant such as polyoxyethylene sorbitan ester employed in the above-described rinse solution. The incubation is continued for sufficient time to permit the sandwiching antibody to bind with exposed fetal restricted antigen epitopes, if any, adhering to the insoluble support. The preferred incubation times and temperatures are as set forth above for the binding of insolubilized reagent anti-(fetal restricted antigen) antibody with the test sample fetal restricted antigen.

The sandwiching antibody solution is then removed from the insoluble support, and the support is rinsed with a rinse solution such as described above, to remove any residual, unbound material.

If the sandwiching antibody is unlabeled, an enzyme labeled antibody or other binding agent which binds selectively with the sandwiching antibody is applied to the insoluble support in an aqueous solution. The solution preferably contains suitable salts and buffers to preserve the reactants and facilitate the binding reaction, such as described above. The incubation is continued for sufficient time to permit labeled anti-(sandwiching antibody) antibody to bind with exposed sandwiching antibody epitopes, if any, adhering to the insoluble support. The preferred incubation times and temperatures are as set forth for the binding of insolubilized reagent anti-(fetal restricted antigen) antibody with the test sample fetal restricted antigen.

The labeled antibody solution is then removed from the insoluble support, and the support is rinsed with a rinse solution such as described above, to remove any residual, unbound labeled material.

In a next step, the insoluble support is contacted with an aqueous solution of a substrate which undergoes a reaction in the presence of the enzyme to release fluorescent or chromogen compound into the solution. Suitable substrates and the enzymes which they can be converted are described in U.S. Patents 4,190,496 and 4,528,267, for example. The support is contacted with an aqueous solution of the substrate containing from $10^{-2}$ to $10^{-10}$ molar concentrations of the substrate. Substrate molar concentrations of from $10^{-4}$ to $10^{-5}$ are preferred. Preferred additional reagents and buffers in the substrate solution include 2-amino-2-methyl-1-propanol buffer and magnesium chloride, for example.

The substrate solution is incubated with the insoluble support for sufficient time for the reaction yielding the fluorophore or chromophore to occur. At temperatures of from 18 to 40°C, incubation times of from 5 to 240 minutes can be used. Preferably, the temperature is within the range of from 20 to 26°C, and the incubation time is from 30 to 90 minutes.

The fluorescent or chromophore level in the solution is then measured. The equipment and procedures for determining the level of fluorescence or chromophore level in the substrate solutions are those conventionally used in the art. The level of fluorescence or chromogen in solution is a function of the enzyme concentration on the insoluble support which is, in turn, a function of the amount of fetal restricted antigen in the test sample. The concentration of the fetal restricted antigen can be determined by comparing the fluorescence or chromophore level of the solution with respective fluorescence or chromophore levels obtained with control solutions containing known concentrations of fetal restricted antigen.

The sandwich procedure can be modified to use a fetal restricted antigen class binding antibody as either the capture or sandwiching antibody. In these embodiments, an anti-(fetal restricted antigen class) antibody, such as an anti-(fibronectin) antibody, is adhered to the insoluble support, and a labeled or unlabeled anti-(fetal restricted antigen) antibody is applied as the sandwiching antibody. Alternatively, the anti-(fetal restricted antigen) antibody can be adhered to the insoluble support, and a labeled or unlabeled anti-(fetal restricted antigen class) antibody is used to sandwich the captured antigen.

Membrane Immunoassays: In a membrane embodiment of this invention to determine fetal restricted antigen in a test sample, an insoluble support to which anti-(fetal restricted antigen) antibody is adhered is contacted with a test sample diluted with an aqueous buffer solution such as phosphate buffer solution (PBS), pH 6 to 8 and preferably from 7.2 to 7.6, for a sufficient time to permit binding of fetal restricted antigen in the sample with the anti-(fetal restricted antigen) antibody on the insoluble support. The time required for binding is very small in a flow through system. Suitable incubation times can be one second up to 20 minutes at temperatures within the range of from 16 to 40°C, the preferred contact time being less than one minute and optimally from 10 seconds to 2 minutes.

The insoluble support is then contacted with an antibody which will bind with the captured fetal restricted antigen on the insoluble support, i.e., the sandwiching antibody. The sandwiching antibody can be labeled or unlabeled. In the event that an unlabeled sandwiching antibody is used, a tertiary antibody which binds with the sandwiching antibody and which bears a physically detectable label can be used in a conventional manner to determine the sandwiching antibody.

A variety of labels are described herein. For purposes of clarity and not by way of limitation, the subsequent steps of the process will be described for anti-(fetal restricted antigen) antibodies which have been labeled with an enzyme, preferably a fluorogenic or chromogenic enzyme.

The sandwiching antibody is applied to the insoluble support in an aqueous solution. The solution preferably contains suitable salts and buffers to preserve the reactants and facilitate the binding reaction. For example, the solution can contain bovine serum albumin (BSA), phosphate buffer solution (PBS), and a mild surfactant such as polyoxyethylene sorbitan ester employed in the above-described rinse solution. The incubation is continued for sufficient time to permit the sandwiching antibody to bind with exposed fetal restricted antigen epitopes, if any, adhering to the insoluble support. The preferred incubation times and temperatures are as set forth for the binding of insolubilized reagent anti-(fetal restricted antigen) antibody with the test sample fetal restricted antigen.

The sandwiching antibody solution optionally can be removed from the insoluble support, and the support is rinsed with a rinse solution such as described above, to remove any residual, unbound labeled material.

If the sandwiching antibody is unlabeled, an enzyme labeled antibody or other binding agent which binds selectively with the sandwiching antibody is applied to the insoluble support in an aqueous solution. The solution preferably contains suitable salts and buffers to preserve the reactants and facilitate the binding reaction. For example, the solution can contain bovine serum albumin (BSA), phosphate buffer solution (PBS), and a mild surfactant such as polyoxyethylene sorbitan ester employed in the above-described rinse solution. The incubation is continued for sufficient time to permit labeled anti-(sandwiching antibody) antibody to bind with sandwiching antibody epitopes, if any, adhering to the insoluble support. The preferred incubation times and temperatures are as set forth for the binding of insolubilized reagent anti-(fetal restricted antigen) antibody with the test sample fetal restricted antigen.

The labeled antibody solution is then removed from the insoluble support, and the support is rinsed with a rinse solution such as described above, to remove any residual, unbound labeled material.

In a next step of the membrane sandwich process of this invention, the insoluble support is contacted with an aqueous solution of a substrate which undergoes a reaction in the presence of the enzyme to release fluorogen or chromogen compound into the solution. Suitable substrates and the enzymes which which they can be converted, as well as additional components and buffers have been described above.

The substrate solution is incubated with the insoluble support for sufficient time for the reaction yielding the fluorophore or chromophore to occur. At temperatures of from 18 to 40°C, incubation times of from 1 to 20 minutes can be used. Preferably, the temperature is within the range of from 20 to 26°C, and the incubation time is from 2 to 5 minutes. The fluorogen or chromogen level on the membrane can be measured using a reflectometer or densitometer.

Competition Immunoassays: Competition embodiments of this invention using labeled reagent fetal restricted antigen comprise contacting a mixture of the test sample and the labeled reagent fetal restricted antigen with an anti-(fetal restricted antigen) antibody adhered to an insoluble support, and determining the amount of label which either binds with the insoluble support or remains in the solution phase.

The competition embodiments of this invention using labeled anti-(fetal restricted antigen) antibodies can be of more than one form. One embodiment using anti-(fetal restricted antigen) antibody bound to the insoluble support comprises contacting a mixture of the test sample and labeled anti-(fetal restricted antigen) antibodies with an anti-(fetal restricted antigen) antibody adhered to an insoluble support, and determining the amount of label which either binds with the insoluble support or remains in the solution phase. Another embodiment using reagent fetal restricted antigen bound to the insoluble support comprises contacting a mixture of the test sample and labeled anti-(fetal restricted antigen) antibodies with a fetal restricted antigen adhered to an insoluble support, and determining the amount of label which either binds with the insoluble support or remains in the solution phase.

In each of these methods, the test sample is diluted with buffer solution, incubated and the label determined as described above with respect to the sandwich immunoassay embodiments. The concentration of the limiting reagent is selected to permit competitive binding between the reagents, with the amount of label remaining on the insoluble support or in the solution being a variable which is a function of the amount of the analyte in the test sample. These methods are generally well known and how to vary them to optimize a procedure are fully within the knowledge of a person skilled in the immunoassay art.

The binding of the anti-(fetal restricted antigen) antibody and the fetal restricted antigen in the test sample can also be determined by agglomeration of particles to which the anti-(fetal restricted antigen) antibody is adhered by fetal restricted antigen in the sample, precipitation of antibodies due to antibody-antigen reactions, or observations of physical or electrical changes which occur upon the antibody-antigen binding, using semiconductor bridge probes, light disturbing patterns such as are described in U.S. Patent 4,647,544, and the like.

A determination of the presence of fetal restricted antigen in the test sample indicates pregnancy.

Fetal Restricted Antigen Pregnancy Test Kits for determining fetal restricted antigen in a test sample included within the scope of this invention generally include (a) an anti-(fetal restricted antigen) antibody, an anti-(fetal restricted antigen class) antibody, or reagent fetal restricted antigen adhered to an insoluble support; (b) a combination of anti-(fetal restricted antigen) antibody adhered to an insoluble support, and either a labeled anti-(fetal restricted antigen) antibody, a labeled anti-(fetal restricted antigen class) antibody, or a labeled reagent fetal restricted antigen; (c) a combination of anti-(fetal restricted antigen class) antibody adhered to an insoluble support together with a labeled anti-(fetal restricted antigen) antibody; and/or (d) a reagent fetal restricted antigen adhered to an insoluble support and a labeled anti-(fetal restricted antigen) antibody.

The kits of this invention can further comprise combinations to determine both of a pregnancy antigen and a fetal restricted antigen in a test sample; buffers for sample transport and storage; vials, foil packages or other containers of reagents of this invention; other, optional reagents such as enzyme substrate reagents in separate vials or other packages; mechanical or optical devices to determine the presence and extent of antibody-antigen binding; and combinations thereof. Sampling devices such as sampling swabs, and buffers for transport and storage can also be included. The individual parts of the kit may be packaged in any convenient form, such as vials, foil packages, or other containers. For example, insoluble support structures in a foil package can be combined with other reagents in vials or other packages.

A method of this invention which determines ectopic pregnancy involves the detection of the presence or absence of a fetal restricted antigen, that is, a uniquely fetal or placental focused material, in a test sample removed in the vicinity of the cervical canal and/or cervical os. Detectable amounts of these materials are normally present in such samples during the first 20 weeks of normal pregnancy. The absence of fetal restricted antigen in such a sample from a pregnant woman during the first 20 weeks of pregnancy is indicative of the presence of an ectopic pregnancy. Since the fetal restricted antigens are not present in significant quantities in the maternal blood, the presence of maternal blood in the sample does not interfere with the test.

The terms "fetal restricted antigen", "fetal restricted antigen class", "antibody", "immunoassay" and "preferential binding" are each defined as described above in reference to the Fetal Restricted Antigen Pregnancy Test.

A test sample which is to be assayed is removed in the vicinity of the cervical canal and/or cervical os, and the sample is assayed to determine the presence or quantity of fetal restricted antigen in the sample. The sample generally comprises fluid and particulate solids, and may contain vaginal or cervical mucus, other vaginal or cervical secretions, cells or cell debris, amniotic fluid, or other fetal or maternal materials. The sample is removed with a swab having a dacron or other fibrous tip, aspirator, suction device, lavage device or the like and is transferred to a suitable container for storage and transport to the testing laboratory.

It is important that the test sample be dispersed in a liquid which preserves the sensitive protein analytes which are unstable in the sampled composition. A suitable storage and transfer medium is that described above in reference to the Fetal Restricted Antigen Pregnancy Test.

Detection of fetal restricted antigen can be achieved by binding the fetal restricted antigen in a test sample with an antibody which binds preferentially with an epitope of the fetal restricted antigen, and determining the presence or absence of this binding.

In one sandwich assay for fetal restricted antigen, the test sample is contacted with an insoluble support to which anti-(fetal restricted antigen) antibody is adhered to effect binding of fetal restricted antigen in the sample to the insoluble support. The insoluble support is then contacted with a secondary antibody, an unlabeled or labeled anti-(fetal restricted antigen) antibody, which binds with the fetal restricted antigen adhering to the insoluble support to label and measure the captured fetal restricted antigen.

An antibody which binds with a class of substances including the analyte fetal restricted antigen can be substituted for the anti-(fetal restricted antigen) antibody capture antibody or the anti-(fetal restricted antigen) antibody sandwiching antibody. For example, anti-(fetal fibronectin) antibody can be adhered to the insoluble support, and labeled or unlabeled anti-(fibronectin) antibody can be used to label the captured antigen. Alternatively, anti-(fibronectin) antibody can be adhered to the insoluble support, and labeled or unlabeled anti-(fetal fibronectin) antibody is used to label the captured antigen. The secondary antibody can have a physically detectable label which can be measured directly on the insoluble support. Alternatively, the secondary antibody can be unlabeled, and the secondary antibody can be determined by contacting the insoluble support with a labeled antibody or antibody fragment which binds selectively with the secondary antibody (i.e., a tertiary antibody), removing unbound labeled tertiary antibody from the support, and determining the presence of the label on the insoluble support. Sandwich immunoassays using a membrane substrate are appropriate for use.

The sample can also be tested by a competition immunoassay procedure. The test sample is mixed with labeled reagent antibody or antigen and incubated with an insoluble support to which an anti-(fetal restricted antigen) antibody or reagent fetal restricted antigen is adhered, competition occurring between the reagents for binding with the sample analyte. Methods and procedures to accomplish such immunoassays are well known to those skilled in the immunoassay art. The label ultimately adhering to the insoluble support or remaining in the solution is determined.

Anti-(fetal restricted antigen) antibody can be obtained from fetal restricted antigens, preferably from highly purified fetal restricted antigens, by conventional antiserum or monoclonal techniques. This invention will be described herein with respect to the detection of fetal fibronectin as a fetal restricted antigen, for purposes of clarity, and not by way of limitation: the detection of any fetal restricted antigen is intended to be within the scope of this invention. Fetal fibronectin is purified from amniotic fluid as described by Engvall and Ruoslahti, *Int.J.Cancer.* **20**:1-5 (1977). Anti-(fetal fibronectin) antibody can be derived from fetal fibronectin by conventional antiserum techniques or by monoclonal antibody techniques.

Both monoclonal and polyclonal anti-(fetal restricted antigen) antibodies, or anti-(fetal restricted antigen class) antibodies can be derived directly from fetal restricted antigens, preferably from highly purified antigens, by conventional antiserum or monoclonal techniques. Antibodies and antibody fragments which are useful in the ectopic pregnancy assays herein, together with their preparation, have been described above for the Fetal Restricted Antigen Pregnancy Test.

The preparation of polyclonal and monoclonal anti-(fetal restricted antigen) antibodies has been described above for the Fetal Restricted Antigen Pregnancy Test, and such preparation methods are appropriate to produce antibodies for use with the Ectopic Pregnancy Test herein.

Immunization protocols, affinity chromatography methods, elution, purification, and concentration of anti-(fetal restricted antigen) antibodies such as anti-(fetal fibronectin) antibodies, and anti-(fetal restricted antigen class) antibodies such as anti-(fibronectin) antibodiesdescribed above with reference to the Fetal Restricted Antigen Pregnancy Test can be used to produce antibodies for use in the Ectopic Pregnancy Test.

Sandwich Immunoassays: In a sandwich embodiment of this invention to determine fetal restricted antigen in a test sample, an insoluble support to which anti-(fetal restricted antigen) antibody is adhered is contacted with a test sample diluted with an aqueous buffer solution, as described above in relation to the Fetal Restricted Antigen Pregnancy Test. Sample diluent, reagent contact, and incubation and rinse conditions, solutions, time and temperature, are as described above in reference to the Fetal Restricted Antigen Pregnancy Test. Residual sample solution is removed from the support by use of a rinse solution, and contacted with a sandwiching antibody under the conditions described above in reference to the Fetal Restricted Antigen Pregnancy Test. The sandwiching antibody solution is then removed from the insoluble support, and the support is rinsed with a rinse solution such as described above, to remove any residual, unbound material, as described.

If the sandwiching antibody is unlabeled, an antibody or other binding agent which binds selectively with the sandwiching antibody is applied to the insoluble support in an aqueous solution, as described above. The labeled antibody solution is then removed from the insoluble support, and the support is rinsed with a rinse solution such as described above, to remove any residual, unbound labeled material. The label is then determined. When the label is an enzyme, as exemplified above, the support is contacted with an appropriate aqueous substrate solution and incubated to produce a fluorophore or chromophore. The fluorescent or chromophore level in the solution is then measured, as described above.

The sandwich procedure can be modified to use a fetal restricted antigen class binding antibody as either the capture or sandwiching antibody, as described above with reference to the Fetal Restricted Antigen Pregnancy Test.

Membrane Immunoassays: In a membrane embodiment to determine fetal restricted antigen in a test sample, an insoluble support to which anti-(fetal restricted antigen) antibody is adhered is contacted with a diluted test sample and incubated under conditions set forth above in reference to the Fetal Restricted Antigen Pregnancy Test.

The insoluble support is then contacted with an antibody which will bind with the captured fetal restricted antigen on the insoluble support, i.e., the sandwiching antibody, as described above. The sandwiching antibody is applied to the insoluble support in an aqueous solution, as described above in reference to the Fetal Restricted Antigen Pregnancy Test. The sandwiching antibody is optionally removed, and the support rinsed, as described above.

If the sandwiching antibody is unlabeled, an enzyme labeled antibody or other binding agent which binds selectively with the sandwiching antibody is applied to the insoluble support in an aqueous solution, as described. Preferred incubation times and temperatures are as set forth above.

The labeled antibody solution is then removed from the insoluble support, and the support is rinsed with a rinse solution such as described above, to remove any residual, unbound labeled material. When the label is an enzyme, as described above, the insoluble support is then contacted with an aqueous solution of a substrate which undergoes a reaction in the presence of the enzyme to release fluorogen or chromogen compound into the solution. Suitable substrates and the enzymes which which they can be converted, as well as additional components and buffers have been described above. The substrate solution is incubated with the insoluble support for sufficient time for the reaction yielding the fluorophore or chromophore to occur, as described above. The fluorogen or chromogen level on the membrane can be measured using a reflectometer or densitometer.

Competition Immunoassays: Competition embodiments of this invention using labeled reagent fetal restricted antigen comprise contacting a mixture of the test sample and the labeled reagent fetal restricted antigen with an anti-(fetal restricted antigen) antibody adhered to an insoluble support, and determining the amount of label which either binds with the insoluble support or remains in the solution phase.

The competition embodiments of this invention using labeled anti-(fetal restricted antigen) antibodies can be of more than one form, as described above with reference to the Fetal Restricted Antigen Pregnancy Test. In each of these methods, the test sample is diluted with buffer solution, incubated and the label determined as described above with respect to the sandwich immunoassay embodiments. The concentration of the limiting reagent is selected to permit competitive binding between the reagents, with the amount of label remaining on the insoluble support or in the solution being a variable which is a function of the amount of the analyte in the test sample. These methods are generally well known and how to vary them to optimize a procedure are fully within the knowledge of a person skilled in the immunoassay art.

The binding of the anti-(fetal restricted antigen) antibody and the fetal restricted antigen in the test sample can also be determined by agglomeration of particles to which the anti-(fetal restricted antigen) antibody is adhered by fetal restricted antigen in the sample, precipitation of antibodies due to antibody-antigen reactions, or observations of physical or electrical changes which occur upon the antibody-antigen binding, using semiconductor bridge probes, light disturbing patterns such as are described in U.S. Patent 4,647,544, and the

EP 0 316 919 B1

like.

It may be desirable to determine pregnancy using the presence of pregnancy-indicating hormones in serum or urine. A wide variety of methods are known to a person skilled in the art for determining the levels of non-restricted pregnancy indicating antigens in the blood or urine of a patient. Any reliable method can be used. Procedures for measuring hCG in plasma, serum and/or urine are described in U.S. Patents 3,171,783, 3,234,096, 3,236,732, 3,298,787, 3,309,275, 3,485,751, 3,655,838, 3,689,633, 3,862,302, 3,873,682, 3,873,683, 3,833,304, 3,991,175, 4,003,988, 4,014,653, 4,016,250, 4,033,723, 4,071,314, 4,094,963, 4,123,224, 4,123,509, 4,138,214, 4,208,187, 4,210,723, 4,234,561, 4,256,629, 4,268,435, 4,270,923, 4,310,455, 4,313,871, 4,320,111, 4,348,207, 4,371,515, 4,419,453, 4,421,896, 4,493,793, 4,508,829, and 4,665,034, for example. Pregnancy detection by measuring progesterone metabolites in urine (U.S. Patent 3,141,740) or in milk, serum or plasma (Hungary Patent No. T37028, WPI No. 86-023344/04); human placental lactogen in serum or plasma (U.S. Patent 3,892,841, 4,371,515, and 4,493,793); estrogen steroids in urine (U.S. Patent 3,955,928); luteinizing hormone (LH), prolactin (PRL) and/or hCG-like substances in serum, plasma or urine (U.S. Patents 4,016,250, 4,094,963, and 4,320,111); pregnancy specific $\beta_1$-glycoprotein (U.S. Patents 4,065,445 and 4,191,533); LH (U.S. Patents 4,138,214 and 4,208,187); bovine pregnancy antigen in bovine serum or urine (European Patent Application 188,551, WPI No. 86-042108/06); a new placental protein (U.S. Patent 4,592,863); and early pregnancy factor WO 8605498 (WPI No. 86-264940/40) have been described. Methods have been described for determining pregnancy by adding dyes to urine (U.S. Patent 2,587,221 and 3,226,196, dinitrophenylhydrazine; U.S. Patent 3,595,620, bromocresol purple or chlorophenol red), by an iodine-paper test (U.S. Patent 3,248,173), by adding other precipitating agents (U.S. Patent 3,278,270), by a treatment of female blood with a mixture of acids and sodium chloride (U.S. Patent 3,883,304). Pregnancy may be determined using an assay of a test sample removed in the vicinity of the cervical canal or cervical os following the teachings of U.S. Application Serial No. 121,902 filed November 17, 1987. Any one of the above methods can be used, but methods such as hCG measurements are preferred.

A determination of the presence of pregnancy together with a negative determination of fetal restricted antigen in the test sample during the first 20 weeks of pregnancy indicates the absence of normal uterine pregnancy, and is an indication that an ectopic pregnancy has occurred.

Ectopic pregnancy kits for determining fetal restricted antigen in a test sample included within the scope of this invention generally include (a) an anti-(fetal restricted antigen) antibody, an anti-(fetal restricted antigen class) antibody, or reagent fetal restricted antigen adhered to an insoluble support; (b) a combination of anti-(fetal restricted antigen) antibody adhered to an insoluble support, and either a labeled anti-(fetal restricted antigen) antibody, a labeled anti-(fetal restricted antigen class) antibody, or a labeled reagent fetal restricted antigen; (c) a combination of anti-(fetal restricted antigen class) antibody adhered to an insoluble support together with a labeled anti-(fetal restricted antigen) antibody; and/or (d) a reagent fetal restricted antigen adhered to an insoluble support and a labeled anti-(fetal restricted antigen) antibody.

The kits to determine ectopic pregnancy can further comprise combinations to determine both a nonrestricted pregnancy antigen and a fetal restricted antigen in a test sample; buffers for sample transport and storage; vials, foil packages or other containers of reagents of this invention; other, optional reagents such as enzyme substrate reagents in separate vials or other packages; mechanical or optical devices to determine the presence and extent of antibody-antigen binding; and combinations thereof. Sampling devices such as sampling swabs, and buffers for transport and storage can also be included. The individual parts of the kit may be packaged in any convenient form, such as vials, foil packages, or other containers. For example, insoluble support structures in a foil package can be combined with other reagents in vials or other packages.

**EX VIVO PRODUCTS OF CONCEPTION TEST**

An embodiment of this invention which determines the presence of *ex vivo* products of conception involves the detection of a fetal restricted antigen, that is, a uniquely fetal or placental focused material, in a test sample expelled by a suspected spontaneous abortion or removed during a dilation and curettage or a therapeutic abortion procedure. Since the fetal restricted antigens are not present in significant quantities in maternal blood, the presence of maternal blood in the sample does not interfere with the tests.

The terms "fetal restricted antigen", "fetal restricted antigen class", "antibody", "immunoassay" and "preferential binding" are each defined as described above in reference to the Fetal Restricted Antigen Pregnancy Test.

A test sample which is to be assayed for the presence of *ex vivo* products of conception is procured which is believed to represent material expulsed from the uterus. Such materials can be tissues removed during a therapeutic abortion or a D&C (dilation and curettage). Alternatively, the material may be vaginal discharge which is believed to be indicative of a spontaneous abortion, or miscarriage. The sample generally comprises

16

fluid and particulate solids, and may contain tissue matter, vaginal or cervical mucus, other vaginal or cervical secretions, cells or cell debris, amniotic fluid, or other fetal or maternal materials. The sample can be procured from the vaginal cavity with a swab having a dacron or other fibrous tip, aspirator, suction device, lavage device or the like. The sample can represent tissues removed during a dilation and curettage procedure or a therapeutic abortion, or can be procured using a feminine protection pad in the case of a suspected spontaneous abortion.

It is important that the test sample be dispersed in a liquid which preserves the sensitive protein analytes which are unstable in the sampled composition. A suitable storage and transfer medium is that described above in reference to the Fetal Restricted Antigen Pregnancy Test.

Detection of fetal restricted antigen can be achieved by binding the fetal restricted antigen in a test sample with an antibody which binds preferentially with an epitope of the fetal restricted antigen, and determining the presence or absence of this binding.

In one sandwich assay for fetal restricted antigen, the test sample is contacted with an insoluble support to which anti-(fetal restricted antigen) antibody is adhered to effect binding of fetal restricted antigen in the sample to the insoluble support. The insoluble support is then contacted with a secondary antibody, an unlabeled or labeled anti-(fetal restricted antigen) antibody, which binds with the fetal restricted antigen adhering to the insoluble support to label and measure the captured fetal restricted antigen.

An antibody which binds with a class of substances including the analyte fetal restricted antigen can be substituted for the anti-(fetal restricted antigen) antibody capture antibody or the anti-(fetal restricted antigen) antibody sandwiching antibody. For example, anti-(fetal fibronectin) antibody can be adhered to the insoluble support, and labeled or unlabeled anti-(fibronectin) antibody can be used to label the captured antigen. Alternatively, anti-(fibronectin) antibody can be adhered to the insoluble support, and labeled or unlabeled anti-(fetal fibronectin) antibody is used to label the captured antigen. The secondary antibody can have a physically detectable label which can be measured directly on the insoluble support. Alternatively, the secondary antibody can be unlabeled, and the secondary antibody can be determined by contacting the insoluble support with a labeled antibody or antibody fragment which binds selectively with the secondary antibody (i.e., a tertiary antibody), removing unbound labeled tertiary antibody from the support, and determining the presence of the label on the insoluble support. Sandwich immunoassays using a membrane substrate are appropriate for use.

The sample can also be tested by a competition immunoassay procedure. The test sample is mixed with labeled reagent antibody or antigen and incubated with an insoluble support to which an anti-(fetal restricted antigen) antibody or reagent fetal restricted antigen is adhered, competition occurring between the reagents for binding with the sample analyte. Methods and procedures to accomplish such immunoassays are well known to those skilled in the immunoassay art. The label ultimately adhering to the insoluble support or remaining in the solution is determined.

Anti-(fetal restricted antigen) antibody can be obtained from fetal restricted antigens, preferably from highly purified fetal restricted antigens, by conventional antiserum or monoclonal techniques. This invention will be described herein with respect to the detection of fetal fibronectin as a fetal restricted antigen, for purposes of clarity, and not by way of limitation: the detection of any fetal restricted antigen is intended to be within the scope of this invention. Fetal fibronectin is purified from amniotic fluid as described by Engvall and Ruoslahti, *Int.J.Cancer.* **20**:1-5 (1977). Anti-(fetal fibronectin) antibody can be derived from fetal fibronectin by conventional antiserum techniques or by monoclonal antibody techniques.

Both monoclonal and polyclonal anti-(fetal restricted antigen) antibodies, or anti-(fetal restricted antigen class) antibodies can be derived directly from fetal restricted antigens, preferably from highly purified antigens, by conventional antiserum or monoclonal techniques. Antibodies and antibody fragments which are useful in the *ex vivo* products of conception assays herein, together with their preparation, have been described above for the Fetal Restricted Antigen Pregnancy Test.

The preparation of polyclonal and monoclonal anti-(fetal restricted antigen) antibodies has been described above for the Fetal Restricted Antigen Pregnancy Test, and such preparation methods are appropriate to produce antibodies for use with the *Ex Vivo* Products of Conception Test herein.

Immunization protocols, affinity chromatography methods, elution, purification, and concentration of anti-(fetal restricted antigen) antibodies such as anti-(fetal fibronectin) antibodies, and anti-(fetal restricted antigen class) antibodies such as anti-(fibronectin) antibodies described above with reference to the Fetal Restricted Antigen Pregnancy Test can be used to produce antibodies for use in the *Ex Vivo* Products of Conception Test herein.

Sandwich Immunoassays: In a sandwich embodiment of this invention to determine fetal restricted antigen in a test sample, an insoluble support to which anti-(fetal restricted antigen) antibody is adhered is contacted with a test sample diluted with an aqueous buffer solution, as described above in relation to the Fetal Restricted Antigen Pregnancy Test. Sample diluent, reagent contact, and incubation and rinse conditions, solutions, time and temperature, are as described above in reference to the Fetal Restricted Antigen Pregnancy Test. Residual sample solution is removed from the support by use of a rinse solution, and contacted with a sandwiching antibody under the conditions described above in reference to the Fetal Restricted Antigen Pregnancy Test. The sandwiching antibody solution is then removed from the insoluble support, and the support is rinsed with a rinse solution such as described above, to remove any residual, unbound material, as described.

If the sandwiching antibody is unlabeled, an antibody or other binding agent which binds selectively with the sandwiching antibody is applied to the insoluble support in an aqueous solution, as described above. The labeled antibody solution is then removed from the insoluble support, and the support is rinsed with a rinse solution such as described above, to remove any residual, unbound labeled material. The label is then determined. When the label is an enzyme, as exemplified above, the support is contacted with an appropriate aqueous substrate solution and incubated to produce a fluorophore or chromophore. The fluorescent or chromophore level in the solution is then measured, as described above.

The sandwich procedure can be modified to use a fetal restricted antigen class binding antibody as either the capture or sandwiching antibody, as described above with reference to the Fetal Restricted Antigen Pregnancy Test.

Membrane Immunoassays: In a membrane embodiment to determine fetal restricted antigen in a test sample, an insoluble support to which anti-(fetal restricted antigen) antibody is adhered is contacted with a diluted test sample and incubated under conditions set forth above in reference to the Fetal Restricted Antigen Pregnancy Test.

The insoluble support is then contacted with an antibody which will bind with the captured fetal restricted antigen on the insoluble support, i.e., the sandwiching antibody, as described above. The sandwiching antibody is applied to the insoluble support in an aqueous solution, as described above in reference to the Fetal Restricted Antigen Pregnancy Test. The sandwiching antibody is optionally removed, and the support rinsed, as described above.

If the sandwiching antibody is unlabeled, an enzyme labeled antibody or other binding agent which binds selectively with the sandwiching antibody is applied to the insoluble support in an aqueous solution, as described. Preferred incubation times and temperatures are as set forth above.

The labeled antibody solution is then removed from the insoluble support, and the support is rinsed with a rinse solution such as described above, to remove any residual, unbound labeled material. When the label is an enzyme, as described above, the insoluble support is then contacted with an aqueous solution of a substrate which undergoes a reaction in the presence of the enzyme to release fluorogen or chromogen compound into the solution. Suitable substrates and the enzymes which they can be converted, as well as additional components and buffers have been described above. The substrate solution is incubated with the insoluble support for sufficient time for the reaction yielding the fluorophore or chromophore to occur, as described above. The fluorogen or chromogen level on the membrane can be measured using a reflectometer or densitometer.

Competition Immunoassays: Competition embodiments of this invention using labeled reagent fetal restricted antigen comprise contacting a mixture of the test sample and the labeled reagent fetal restricted antigen with an anti-(fetal restricted antigen) antibody adhered to an insoluble support, and determining the amount of label which either binds with the insoluble support or remains in the solution phase.

The competition embodiments of this invention using labeled anti-(fetal restricted antigen) antibodies can be of more than one form, as described above with reference to the Fetal Restricted Antigen Pregnancy Test. In each of these methods, the test sample is diluted with buffer solution, incubated and the label determined as described above with respect to the sandwich immunoassay embodiments. The concentration of the limiting reagent is selected to permit competitive binding between the reagents, with the amount of label remaining on the insoluble support or in the solution being a variable which is a function of the amount of the analyte in the test sample. These methods are generally well known and how to vary them to optimize a procedure are fully within the knowledge of a person skilled in the immunoassay art.

The binding of the anti-(fetal restricted antigen) antibody and the fetal restricted antigen in the test sample can also be determined by agglomeration of particles to which the anti-(fetal restricted antigen) antibody is adhered by fetal restricted antigen in the sample, precipitation of antibodies due to antibody-antigen reactions, or observations of physical or electrical changes which occur upon the antibody-antigen binding, using semiconductor bridge probes, light disturbing patterns such as are described in U.S. Patent 4,647,544, and the like.

It may be desirable to determine pregnancy using the presence of pregnancy-indicating hormones in serum or urine. A wide variety of methods are known to a person skilled in the art for determining the levels of non-restricted pregnancy indicating antigens in the blood or urine of a patient. Any reliable method can be used, such as those described above with reference to the Ectopic Pregnancy Test.

A test sample obtained during a D&C, therapeutic abortion, or suspected spontaneous abortion (miscarriage) can be tested for the presence of fetal restricted antigen. Concurrent determinations of the presence of pregnancy, and negative determination of fetal restricted antigen in a test sample suspected of representing a therapeutic or spontaneous abortion, is an indication that pregnancy has occurred, and is continuing. Determination of the presence of pregnancy, and positive determination of fetal restricted antigen in a test sample suspected of representing a therapeutic or spontaneous abortion is an indication that pregnancy has occurred, but has been terminated. Determination of the absence of both of pregnancy indicators, and negative determination of fetal restricted antigen in a test sample derived from a D&C is an indication that pregnancy has not occurred, and that no pregnancy has been terminated.

*Ex vivo* Products of Conception Test Kits for determining fetal restricted antigen in a test sample included within the scope of this invention generally include (a) an anti-(fetal restricted antigen) antibody, an anti-(fetal restricted antigen class) antibody, or reagent fetal restricted antigen adhered to an insoluble support; (b) a combination of anti-(fetal restricted antigen) antibody adhered to an insoluble support, and either a labeled anti-(fetal restricted antigen) antibody, a labeled anti-(fetal restricted antigen class) antibody, or a labeled reagent fetal restricted antigen; (c) a combination of anti-(fetal restricted antigen class) antibody adhered to an insoluble support together with a labeled anti-(fetal restricted antigen) antibody; and/or (d) a reagent fetal restricted antigen adhered to an insoluble support and a labeled anti-(fetal restricted antigen) antibody.

The kits to determine *ex vivo* products of conception can further comprise combinations to determine both a nonrestricted pregnancy antigen and a fetal restricted antigen in a test sample; buffers for sample transport and storage; vials, foil packages or other containers of reagents of this invention; other, optional reagents such as enzyme substrate reagents in separate vials or other packages; mechanical or optical devices to determine the presence and extent of antibody-antigen binding; and combinations thereof. Sampling devices such as sampling swabs, and buffers for transport and storage can also be included. The individual parts of the kit may be packaged in any convenient form, such as vials, foil packages, or other containers. For example, insoluble support structures in a foil package can be combined with other reagents in vials or other packages.

## RISK OF PRETERM LABOR / RUPTURED MEMBRANE TEST

An embodiment of this invention for indicating increased risk of preterm birth involves the detection of a fetal restricted antigen in a test sample after week 20 of pregnancy. We have discovered that detectable amounts of these materials are generally absent from vaginal samples such as those obtained from the vicinity of the posterior fornix, cervical canal or cervical os after week 20 of pregnancy. Detectable amounts of these materials present in such samples taken after week 20 pregnancy indicate an increased risk of impending preterm birth and/or indicate the rupture of the amniotic membrane. Since the fetal restricted antigens are not present in significant quantities in the maternal blood, the presence of maternal blood in the sample does not interfere with the test.

The terms "fetal restricted antigen", "fetal restricted antigen class", "antibody", "immunoassay" and "preferential binding" are each defined as described above in reference to the Fetal Restricted Antigen Pregnancy Test.

A test sample which is to be assayed is removed from the vicinity of the posterior fornix, cervical canal or cervical os, and the sample is assayed to determine the presence or quantity of fetal restricted antigen in the sample. The sample generally comprises fluid and particulate solids, and may contain vaginal or cervical mucus, other vaginal or cervical secretions, cells or cell debris, amniotic fluid, or other fetal or maternal materials. The sample is removed with a swab having a dacron or other fibrous tip, aspirator, suction device, lavage device or the like and is transferred to a suitable container for storage and transport to the testing laboratory.

It is important that the test sample be dispersed in a liquid which preserves the sensitive protein analytes which are unstable in the sampled composition. A suitable storage and transfer medium is that described above in reference to the Fetal Restricted Antigen Pregnancy Test.

Detection of fetal restricted antigen can be achieved by binding the fetal restricted antigen in a test sample with an antibody which binds preferentially with an epitope of the fetal restricted antigen, and determining the presence or absence of this binding.

In one sandwich assay for fetal restricted antigen, the test sample is contacted with an insoluble support to which anti-(fetal restricted antigen) antibody is adhered to effect binding of fetal restricted antigen in the sample to the insoluble support. The insoluble support is then contacted with a secondary antibody, an unlabeled or labeled anti-(fetal restricted antigen) antibody, which binds with the fetal restricted antigen adhering to the insoluble support to label and measure the captured fetal restricted antigen.

An antibody which binds with a class of substances including the analyte fetal restricted antigen can be substituted for the anti-(fetal restricted antigen) antibody capture antibody or the anti-(fetal restricted antigen) antibody sandwiching antibody. For example, anti-(fetal fibronectin) antibody can be adhered to the insoluble support, and labeled or unlabeled anti-(fibronectin) antibody can be used to label the captured antigen. Alternatively, anti-(fibronectin) antibody can be adhered to the insoluble support, and labeled or unlabeled anti-(fetal fibronectin) antibody is used to label the captured antigen. The secondary antibody can have a physically detectable label which can be measured directly on the insoluble support. Alternatively, the secondary antibody can be unlabeled, and the secondary antibody can be determined by contacting the insoluble support with a labeled antibody or antibody fragment which binds selectively with the secondary antibody (i.e., a tertiary antibody), removing unbound labeled tertiary antibody from the support, and determining the presence of the label on the insoluble support. Sandwich immunoassays using a membrane substrate are appropriate for use.

The sample can also be tested by a competition immunoassay procedure. The test sample is mixed with labeled reagent antibody or antigen and incubated with an insoluble support to which an anti-(fetal restricted antigen) antibody or reagent fetal restricted antigen is adhered, competition occurring between the reagents for binding with the sample analyte. Methods and procedures to accomplish such immunoassays are well known to those skilled in the immunoassay art. The label ultimately adhering to the insoluble support or remaining in the solution is determined.

Anti-(fetal restricted antigen) antibody can be obtained from fetal restricted antigens, preferably from highly purified fetal restricted antigens, by conventional antiserum or monoclonal techniques. This invention will be described herein with respect to the detection of fetal fibronectin as a fetal restricted antigen, for purposes of clarity, and not by way of limitation: the detection of any fetal restricted antigen is intended to be within the scope of this invention. Fetal fibronectin is purified from amniotic fluid as described by Engvall and Ruoslahti, *Int.J.Cancer.* **20**:1-5 (1977). Anti-(fetal fibronectin) antibody can be derived from fetal fibronectin by conventional antiserum techniques or by monoclonal antibody techniques.

Both monoclonal and polyclonal anti-(fetal restricted antigen) antibodies, or anti-(fetal restricted antigen class) antibodies can be derived directly from fetal restricted antigens, preferably from highly purified antigens, by conventional antiserum or monoclonal techniques. Antibodies and antibody fragments which are useful in the assays herein, together with their preparation, have been described above for the Fetal Restricted Antigen Pregnancy Test.

The preparation of polyclonal and monoclonal anti-(fetal restricted antigen) antibodies has been described above for the Fetal Restricted Antigen Pregnancy Test, and such preparation methods are appropriate to produce antibodies for use with the Risk of Preterm Labor / Rupture of Membranes Test herein.

Immunization protocols, affinity chromatography methods, elution, purification, and concentration of anti-(fetal restricted antigen) antibodies such as anti-(fetal fibronectin) antibodies, and anti-(fetal restricted antigen class) antibodies such as anti-(fibronectin) antibodies described above with reference to the Fetal Restricted Antigen Pregnancy Test can be used to produce antibodies for use in the Preterm Labor / Rupture of Membranes Test.

Sandwich Immunoassays: In a sandwich embodiment of this invention to determine fetal restricted antigen in a test sample, an insoluble support to which anti-(fetal restricted antigen) antibody is adhered is contacted with a test sample diluted with an aqueous buffer solution, as described above in relation to the Fetal Restricted Antigen Pregnancy Test. Sample diluent, reagent contact, and incubation and rinse conditions, solutions, time and temperature, are as described above in reference to the Fetal Restricted Antigen Pregnancy Test. Residual sample solution is removed from the support by use of a rinse solution, and contacted with a sandwiching antibody under the conditions described above in reference to the Fetal Restricted Antigen Pregnancy Test. The sandwiching antibody solution is then removed from the insoluble support, and the support is rinsed with a rinse solution such as described above, to remove any residual, unbound material, as described.

If the sandwiching antibody is unlabeled, an antibody or other binding agent which binds selectively with the sandwiching antibody is applied to the insoluble support in an aqueous solution, as described above. The labeled antibody solution is then removed from the insoluble support, and the support is rinsed with a rinse solution such as described above, to remove any residual, unbound labeled material. The label is then determined. When the label is an enzyme, as exemplified above, the support is contacted with an appropriate aqueous substrate solution and incubated to produce a fluorophore or chromophore. The fluorescent or chromophore level in the solution is then measured, as described above.

The sandwich procedure can be modified to use a fetal restricted antigen class binding antibody as either the capture or sandwiching antibody, as described above with reference to the Fetal Restricted Antigen Pregnancy Test.

Membrane Immunoassays: In a membrane embodiment to determine fetal restricted antigen in a test sample, an insoluble support to which anti-(fetal restricted antigen) antibody is adhered is contacted with a diluted test sample and incubated under conditions set forth above in reference to the Fetal Restricted Antigen Pregnancy Test.

The insoluble support is then contacted with an antibody which will bind with the captured fetal restricted antigen on the insoluble support, i.e., the sandwiching antibody, as described above. The sandwiching antibody is applied to the insoluble support in an aqueous solution, as described above in reference to the Fetal Restricted Antigen Pregnancy Test. The sandwiching antibody is optionally removed, and the support rinsed, as described above.

If the sandwiching antibody is unlabeled, an enzyme labeled antibody or other binding agent which binds selectively with the sandwiching antibody is applied to the insoluble support in an aqueous solution, as described. Preferred incubation times and temperatures are as set forth above.

The labeled antibody solution is then removed from the insoluble support, and the support is rinsed with a rinse solution such as described above, to remove any residual, unbound labeled material. When the label is an enzyme, as described above, the insoluble support is then contacted with an aqueous solution of a substrate which undergoes a reaction in the presence of the enzyme to release fluorogen or chromogen compound into the solution. Suitable substrates and the enzymes which which they can be converted, as well as additional components and buffers have been described above. The substrate solution is incubated with the insoluble support for sufficient time for the reaction yielding the fluorophore or chromophore to occur, as described above. The fluorogen or chromogen level on the membrane can be measured using a reflectometer or densitometer.

Competition Immunoassays: Competition embodiments of this invention using labeled reagent fetal restricted antigen comprise contacting a mixture of the test sample and the labeled reagent fetal restricted antigen with an anti-(fetal restricted antigen) antibody adhered to an insoluble support, and determining the amount of label which either binds with the insoluble support or remains in the solution phase.

The competition embodiments of this invention using labeled anti-(fetal restricted antigen) antibodies can be of more than one form, as described above with reference to the Fetal Restricted Antigen Pregnancy Test. In each of these methods, the test sample is diluted with buffer solution, incubated and the label determined as described above with respect to the sandwich immunoassay embodiments. The concentration of the limiting reagent is selected to permit competitive binding between the reagents, with the amount of label remaining on the insoluble support or in the solution being a variable which is a function of the amount of the analyte in the test sample. These methods are generally well known and how to vary them to optimize a procedure are fully within the knowledge of a person skilled in the immunoassay art.

The binding of the anti-(fetal restricted antigen) antibody and the fetal restricted antigen in the test sample can also be determined by agglomeration of particles to which the anti-(fetal restricted antigen) antibody is adhered by fetal restricted antigen in the sample, precipitation of antibodies due to antibody-antigen reactions, or observations of physical or electrical changes which occur upon the antibody-antigen binding, using semiconductor bridge probes, light disturbing patterns such as are described in U.S. Patent 4,647,544, and the like.

A determination of the presence of fetal restricted antigen in a test sample after 20 weeks of pregnancy indicates that fetal membranes may have ruptured, and/or is an indication of increased risk of preterm labor.

Risk of Preterm Labor / Rupture of Membranes Test Kits for determining fetal restricted antigen in a test sample included within the scope of this invention generally include (a) an anti-(fetal restricted antigen) antibody, an anti-(fetal restricted antigen class) antibody, or reagent fetal restricted antigen adhered to an insoluble support; (b) a combination of anti-(fetal restricted antigen) antibody adhered to an insoluble support, and either a labeled anti-(fetal restricted antigen) antibody, a labeled anti-(fetal restricted antigen class) antibody, or a labeled reagent fetal restricted antigen; (c) a combination of anti-(fetal restricted antigen class) antibody adhered to an insoluble support together with a labeled anti-(fetal restricted antigen) antibody; and/or (d) a reagent fetal restricted antigen adhered to an insoluble support and a labeled anti-(fetal restricted antigen) antibody.

The kits can further comprise combinations to determine both a nonrestricted pregnancy antigen and a fetal restricted antigen in a test sample; buffers for sample transport and storage; vials, foil packages or other containers of reagents of this invention; other, optional reagents such as enzyme substrate reagents in separate vials or other packages; mechanical or optical devices to determine the presence and extent of antibody-antigen binding; and combinations thereof. Sampling devices such as sampling swabs, and buffers for transport and storage can also be included. The individual parts of the kit may be packaged in any convenient form, such as vials, foil packages, or other containers. For example, insoluble support structures in a foil package can be combined with other reagents in vials or other packages.

## INSOLUBLE SUPPORT REAGENTS

Antigen and antibody reagents of this invention can be bonded to insoluble supports by conventional processes. Antigen binding methods suitable for binding antigens to insoluble supports such as those described in U.S. Patents 3,234,096, 3,236,732, 3,309,275, 3,873,683, 3,991,175, 4,003,988, 4,016,250, 4,033,723, 4,071,314, 4,348,207, and 4,419,453, for binding antigens to latex particles and erythrocytes, for example, can be used. Procedures for binding of antibodies to insoluble supports are described in U.S. Patents 3,551,555, 3,553,310, 4,048,298 and RE-29,474, and by Tijsson, PRACTICE AND THEORY OF ENZYME IMMUNOASSAYS. Elsevier Science Publishers, (1985) pp 297-328, for example. Procedures for binding of antibodies to polystyrene by adsorption are described in U.S. Patents 3,646,346 and 4,092,408, for example. For purposes of clarity and not by way of limitation, the binding procedures are described herein with respect to the binding of antibodies to insoluble supports. These procedures are suitable for binding of reagent antibodies, such as anti-(pregnancy antigen) antibodies, anti-(fetal restricted antigen) antibodies, and anti-(fetal restricted antigen class) antibodies, and for binding of reagent antigens such as reagent pregnancy antigen or reagent fetal restricted antigen, to insoluble supports.

A variety of materials can be used as the insoluble support, the primary consideration being the binding of the antibody to the surface, and the absence of interference with the antigen binding reaction or with other reactions which can be employed to determine the presence and extent of the binding reaction. Organic and inorganic polymers, both natural and synthetic, can be used as the insoluble support. Examples of suitable polymers include polyethylene, polypropylene, polybutylene, poly(4-methylbutylene), butyl rubber, silastic polymers, polyesters, polyamides, cellulose and cellulose derivatives (such as cellulose acetate, nitrocellulose and the like), acrylates, methacrylates, vinyl polymers (such as polyvinyl acetate, polyvinyl chloride, polyvinylidene chloride, polyvinyl fluoride, and the like), polystyrene and styrene graft copolymers, rayon, nylon, polyvinylbutyrate, polyformaldehyde, etc. Other materials which can be used as the insoluble support can the latexes of the above polymers, silica gel, silicon wafers, glass, paper, insoluble protein, metals, metalloids, metal oxides, magnetic materials, semi-conductive materials, cermets and the like. In addition are included substances which form gels, such as proteins such as gelatins, lipopolysaccharides, silicates, agarose, polyacrylamides or polymers which form several aqueous phases such as dextrans, polyalkylene glycols (alkylene with 2 to 3 carbon atoms) or surfactants, e.g. amphophilic compounds such as phospholipids, long chain (12-24 carbon atoms) alkyl ammonium salts and the like.

A preferred diagnostic support of this invention comprises a nylon or nitrocellulose membrane. Alternate diagnostic supports are made from a polystyrene, styrene copolymers such as styrene-acrylonitrile copolymers, or polyolefins such as polyethylene or polypropylene, and acrylate and methacrylate polymers and copolymers. The reagent antibody or antigen reagents can be bound to the insoluble support by adsorption, ionic bonding, van der Waals adsorption, electrostatic bonding, or other non-covalent bonding, or it can be bound to the insoluble support by covalent bonding. A particularly advantageous support for this procedure comprises a microtiter plate having a plurality of wells. The well surface or plastic cup inserts therein can constitute the antigen or antibody support. If the determination will require the use of fluorometric measurements, the microtiter plate or the well inserts are advantageously opaque to light so that excitation light applied to a well does not reach or influence contents of the surrounding wells.

Procedures for non-covalent bonding are described in U.S. Patent 4,528,267. Procedures for covalently bonding antibodies and antigens to insoluble supports are described by I. Chibata in IMMOBILIZED ENZYMES. Halsted Press: New York (1978) and A. Cuatrecasas, *J.Bio.Chem.* **245**:3059 (1970). The surface can be coated with a protein and coupled with the antibody or antigen using procedures described in U.S. Patent 4,210,418 using glutaraldehyde as a coupling agent, for example. In an alternate procedure, the well can be coated with a layer having free isocyanate groups such as a polyether isocyanate, and application of the antibody or antigen in aqueous solution thereto effects the requisite bonding. In yet another procedure, the antibody or antigen can be coupled to a hydroxylated material by means of cyanogen bromide as described in U.S. Patent 3,720,760.

The insoluble supports are preferably "blocked" to reduce nonspecific binding. The choice of suitable blocking agents is determined by the type of insoluble support. For example, for polystyrene supports, suitable blocking agents include water-soluble non-immune animal proteins. Suitable water-soluble non-immune animal proteins include bovine serum albumin (BSA); human, rabbit, goat, sheep, and horse serum albumins; casein and non-fat milk; ovalbumin; glycoproteins; and the like.

The same blocking agents can also be used for nylon and nitrocellulose supports. However, a preferred blocking agent for nitrocellulose or nylon membrane supports is non-fat milk or casein. An optimum blocking agent for these membrane supports is an aqueous solution containing from 1 to 5 wt.% non-fat dried milk or casein, and nonionic surfactants such as polyoxyethylene sorbitan derivatives and polyoxyethylene ethers.

## LABELED REAGENTS

The labeled reagent pregnancy antigen, anti-(pregnancy antigen) antibody, anti-(sandwiching antibody) reagent antibodies of this invention can be prepared by conventional procedures for attaching labels to proteins, preferably with suitable protection of antibody binding sites.

The labeled reagent fetal restricted antigen, anti-(fetal restricted antigen) antibody, anti-(fetal restricted antigen class) antibody and anti-(sandwiching antibody) reagent antibodies of this invention can be prepared by conventional procedures for attaching labels to proteins, preferably with suitable protection of antibody binding sites.

The labels can be bonded or coupled to the protein reagents by chemical or physical bonding. Ligands and groups which can be conjugated to the reagent antigen or antibodies of this invention include elements, compounds or biological materials which have physical or chemical characteristics which can be used to distinguish the reagents to which they are bonded from compounds and materials in the sample being tested.

Labeling procedures are described herein with respect to labeling antibodies for purposes of clarity and not by way of limitation, and the procedures described are generally suitable for labeling any proteinaceous compound or substance, such as the reagent pregnancy antigens or fetal restricted antigens herein.

Radiolabeled antibodies of this invention can be used for *in vitro* diagnostic tests. The specific activity of a tagged antibody depends upon the half-life, isotopic purity of the radioactive label and how the label is incorporated into the antigen or antibody. Table A lists several commonly used isotopes, their specific activities and half-lives. In immunoassay tests, the higher the specific activity, in general, the better the sensitivity.

## Table A

| Isotope | Specific Activity of Pure Isotope (Curies/mole) | Half-Life |
|---------|--------------------------------------------------|-----------|
| $^{14}C$ | $6.25 \times 10^1$ | 5720 years |
| $^{3}H$ | $2.91 \times 10^4$ | 12.5 years |
| $^{35}S$ | $1.50 \times 10^6$ | 87 days |
| $^{125}I$ | $2.18 \times 10^6$ | 60 days |
| $^{32}P$ | $3.16 \times 10^6$ | 14.3 days |
| $^{131}I$ | $1.62 \times 10^7$ | 8.1 days |

Procedures for labeling antibodies with radioactive isotopes listed in Table A are generally known in the art. Tritium labeling procedures are described in U.S. Patent 4,302,438, for example. Iodinating, tritium labeling and $^{35}S$ labeling procedures especially adapted for antibodies are described by Goding, J., MONOCLONAL ANTIBODIES: PRINCIPLES AND PRACTICE. New York: Academic Press (1983) pp 124-126, and the references cited therein. Other procedures for iodinating antibodies are described by Hunter and Greenwood, *Nature.* **144**:945 (1962) and David et al, *Biochem.* **13**:1014-1021 (1974) and in U.S. Patents 3,867,517 and 4,376,110. Examples of suitable systems, coupling procedures and substrate reactions therewith are disclosed in U.S. Patents RE-31,006, 3,654,090, 4,214,048, 4,289,747, 4,302,438, 4,312,943, 4,376,110 and the references cited therein, for example. Examples of other suitable systems are described by Pesce et al, *Clin.Chem.* **20**:353-359 (1974) and Wisdom, G., *Clin.Chem.* **22**:1243 (1976).

A list of suitable enzyme classes which can be used for labeling, and specific examples for each class, follow:

## Table B

| Class | Enzyme Example |
| --- | --- |
| Hydrolases Carbohydrases | Amylases |
| Nucleases | Polynucleotidase |
| Amidases | Arginase |
| Purine deaminases | Adenase |
| Peptidases | Aminopolypeptidase |
| Proteinases | Pepsin |
| Esterases | Lipases |
| Iron Enzymes | Catalase |
| Copper Enzymes | Tyrosinases |
| Enzymes containing Coenzymes | Alcohol dehydrogenase |
| Enzymes reducing cytochrome | Succinic dehydrogenase |
| Yellow enzymes | Diaphorase |
| Mutases | Glyoxalase |
| Demolases | Aldolase |
| Oxidases | Glucose oxidase |
| | Horseradish peroxidase |

Suitable enzymes are described in Hawk et al, PRACTICAL PHYSIOLOGICAL CHEMISTRY, New York: McGraw-Hill pp 306-397 (1954).

Fluorogenic and chromogenic enzymes (enzymes in the presence of which a selected substrate will produce a fluorescent or chromogenic product) are useful labeling moieties. Methods for selectively conjugating enzymes to antibodies without impairing the ability of the antibody to bind with antigen and for conjugating enzymes to proteinaceous reagents are well known in the art.

Suitable enzymes and procedures for coupling them to antibodies are described by I. Chibata in IMMOBILIZED ENZYMES. Halsted Press: New York (1978); A. Cuatrecasas, *J.Bio.Chem.* **245**:3059 (1970); Wilson, M. et al, INTERNATIONAL CONFERENCE IN IMMUNOFLUORESCENCE AND RELATED STAINING TECHNIQUES. W. Knapp et al, editors. Amsterdam: Elsevier pp 215-244 (1978); Sullivan, M. et al, *Ann.Clin.Biochem.* **16**:221-240 (1979); Nygren, H. et al, *Med.Biol.* **57**:187-191 (1979); Gadkari, D. et al, *J.Virol.Meth.* **10**:215-224 (1985); Tijssen, P. et al, *Anal.Biochem.* **136**:451-457 (1984); Tsuruta, J. et al, *J.Histochem.Cytochem.* **33**:767-777 (1985); Ishikawa, E., *J.Immunoassay.* **4**:209-327 (1983); and in U.S. Patent 4,190,496, for example.

The preferred enzymes and suitable substrates corresponding thereto include horseradish peroxidase for which suitable substrates are o-phenylenediamine, m-phenylenediamine, o-dianisidine, and 4-chloro-α-napthol. They also include β-galactosidase for which suitable substrates are 4-methylumbelliferyl-β-D-galactoside, p-nitrophenyl-β-D-galactose, p-nitrophenol, o-nitrophenyl-β-D-galactose, and o-nitrophenol, for example. They include alkaline phosphatase for which suitable substrates are p-nitrophenylphosphate, indoxyl phosphate, and 5-bromo-3-chloroindoxyl phosphate, for example.

Examples of suitable procedures for enzyme labeling the antibody include the use of carbodiimides, dialdehydes, and bifunctional coupling reagents. Linkage of enzymes through amide groups can be achieved by treating the proteins with thionyl chloride, N-hydroxysuccinimide or similar reagents in an anhydrous solvent such as dimethylformamide, dioxane, dimethylsulfoxide, tetrahydrofuran, or the like. Alternative coupling agents include carbodiimides such as 1-ethyl-3-(3-(N,N′-dimethylamino)propyl)carbodiimide, 1-cyclohexyl-3-(2-morpholinoethyl)carbodiimide methyl-p-toluenesulfonate, succinimidyl 4-(N-maleimidoethyl)-cyclohexane-1-carboxylate, and succinimidyl 3-(2-pyridyldithio)-propionate, for example.

The carbohydrate moiety of an enzyme can also be oxidized to an aldehyde and reacted with lysyl amino groups of immunoglobulins to form a Schiffs base. Reduction with sodium borohydride effects a stable linkage of enzyme and antibody. Horseradish peroxidase with antibody can be efficiently linked to immunoglobulins by the method of Wilson, M. et al, INTERNATIONAL CONFERENCE IN IMMUNOFLUORESCENCE AND RELATED STAINING TECHNIQUES. W. Knapp et al, editors. Amsterdam: Elsevier pp 215-244 (1978).

Fluorophore and chromophore labeled antibodies can be prepared from standard fluorescent moieties known in the art. Since antibodies and other proteins absorb light having wavelengths up to about 310 nm, the fluorescent moieties should be selected to have substantial absorption at wavelengths above 310 nm and preferably above 400 nm. A variety of suitable fluorescers and chromophores are described by Stryer, *Science.* **162**:526 (1968) and Brand, L. et al, *Ann.Rev.Biochem.* **41**:843-868 (1972). The antibodies can be labeled with fluorescent chromophore groups by conventional procedures such as those disclosed in U.S. Patents 3,940,475, 4,289,747 and 4,376,110, for example.

One group of fluorescers having a number of the desirable properties described above are the xanthene dyes, which include the fluoresceins derived from 3,6-dihydroxy-9-phenylxanthhydrol and resamines and rhodamines derived from 3,6-diamino-9-phenylxanthydrol and lissanime rhodamine B. The rhodamine and fluorescein derivatives of 9-*o*-carboxyphenylxanthhydrol have a 9-*o*-carboxyphenyl group. Fluorescein compounds having reactive coupling groups such as amino and isothiocyanate groups such as fluorescein isothiocyanate and fluorescamine are readily available.

Another group of fluorescent compounds are the naphthylamines, having an amino group in the alpha or beta position. Included among the naphthylamino compounds are 1-dimethylaminonaphthyl-5-sulfonate, 1-anilino-8-naphthalene sulfonate and 2-*p*-toluidinyl-6-naphthalene sulfonate. Other dyes include 3-phenyl-7-isocyanatocoumarin; acridines such as 9-isothiocyanatoacridine and acridine orange; N-[*p*-(2-benzoxazolyl)phenyl]maleimide; benzoxadiozoles such as 4-chloro-7-nitrobenzo-2-oxa-1,3-diazole and 7-(*p*-methoxybenzylamino)-4-nitrobenzo-2-oxa-1,3-diazole; stilbenes such as 4-dimethylamino-4′-isothiocyanatostilbene and 4-dimethylamino-4′-maleimidostilbene; N,N′-dioctadecycloxacarboxyamine-p-toluenesulfonate; pyrenes such as 8-hydroxy-1,3,6-pyrenetrisulfonic acid, 1-pyrenebutyric acid, merocyanine 540, rose bengal, 2,4-diphenyl-3(2H)-furanone, *o*-phthaldehyde, as well as other readily available fluorescing molecules. These dyes either have active functionalities or such functionalities can be readily introduced.

Antibodies can be labeled with fluorochromes or chromophores by the procedures described by Goding, J., MONOCLONAL ANTIBODIES: PRINCIPLES AND PRACTICE. New York: Academic Press (1983) pp 208-249. The concentration of fluorochrome is selected according to the table of Goding, supra, p 229. For example, fluorescein isocyanate (1.0 mg/mL) or rhodamine isocyanate (10.0 mg/mL) in DMSO is prepared, and the desired volume (1-10% of total protein solution volume) is added to the protein solution dropwise, with stirring. The reaction proceeds for two hours, shielded from light. The product is purified by gel filtration on SEPHADEX G-25 gel in PBS containing 0.1% $NaNO_3$ to separate the unreacted or hydrolyzed fluorochrome. The absorbence of the conjugate is measured at 280 nm and at its peak in the visible region (495 nm for fluoresceinated antibody and 550 nm for rhodaminated antibody). The fluorochrome to protein ratio is calculated according to the procedure of Goding, MONOCLONAL ANTIBODIES: PRINCIPLES AND PRACTICE. New York: Academic Press (1983) pp 224-225. Conjugates are stored at 4°C protected from light until use. If the antibody solution concentration is less than 1 mg/mL, BSA is added to the solution to a final concentration of 1 mg/mL.

The antibodies and reagent antigens used in the assays of this invention can be covalently bonded to avidin or biotin. Suitable binding procedures involve cross-linking through a bifunctional cross-linking agent. Suitable bifunctional compounds are described by Peters, K. et al, *Ann.Rev.Biochim.* **46**:523 (1977). Alkyl imidates show a high degree of specificity among the functional groups presented to them by a protein. The reaction is specific for primary amino groups. Examples of suitable coupling reagents include amidoesters such as dimethylmalonimidate, azides such as the acyl azide of tartryl diazide which reacts readily with immuno groups to produce amide linkages. Aryl dihalides (e.g., 1,5-difluoro-2,4-dinitrobenzene, or 4,4′-difluoro-3,3′-dinitrophenyl sulfone, glutaraldehyde, 1-ethyl-3-(3-dimethylaminopropyl)carbodiimide hydrochloride, dimaleimide, mixed anhydride, m-maleamidobenzoyl N-hydroxysucciinimide ester, and other known cross-linking agents can be used.

The foregoing reagents provide essentially irreversible bonds. Bifunctional agents with functional groups such as disulfide or glycol may be used. These provide bonds which can be broken after the cross-linking reaction, if desired. Such reagents include dimethyl 3,3′-dithiobispropionimidate, succinimidylpropionimidate, N-(3-fluoro-4,6-dinitrophenyl)-cystamine, tartryl diazide, tartryl di(glycylazide) and tartryl di(epsilon-amino caproylazide).

In other instances, the bonds can be formed directly between the reagents themselves. For example, antibody can be bound to biotin through functional groups on the respective materials. As a specific example, biotin can be treated with periodate and reacted with antibody to give a Schiff base formation without inhibiting the biotin to avidin binding or blocking immunological activity of the antibody. Avidin-conjugated and biotinylated reagents are available from Vector Laboratories, Burlingame, California.

Known techniques using bifunctional cross-linking agents include the following: (a) a one-step glutaraldehyde linkage, Avrameas, S., *Immunochem.* **6**:43 (1969); (b) two-step glutaraldehyde linkage, Avrameas, S., *Immunochem.* **8**:1175 (1971); and (c) dimaleimide linkage, Kato, K. et al, *Euro.J.Biochem.* **62**:285 (1966).

Antibodies can be labeled with metallic radionuclides according the procedure of Hnatowich, D. et al. *J-.Appl.Rad.* **35**:554-557 (1984) and Buckley, R et al. *fed.Eur.Biochem.Soc.* **166**:202-204 (Jan. 1984). In this procedure the antibodies are conjugated with a chelating agent, such as diethylenetriaminepentaacetic acid (DTPA), which is capable of forming a chelate with the metallic radionuclide. A suspension of 0.1 mg/mL of the bicyclic anhydride of DTPA is prepared in a dry solvent such as chloroform, ether or dry DMSO. An aliquot is removed to a clean, dry tube sufficient to provide a DTPA to immunoglobulin molar ratio of 1:1 and evaporated under nitrogen. A 10-20 microliter portion of the antibody solution used (10-20 mg/mL) in 0.05 M bicarbonate buffer in saline, pH 7.0-7.5, is added to the dry DTPA, and the contents are agitated for 0.5-1.0 minute. The coupled protein preparation is diluted to 0.2 mL with the same buffer solution and purified on a 5 cm gel filtration column with SEPHADEX Trade Mark G-50 gel, using a saline eluant. The coupling efficiency is determined before purification by the addition of "chelation-grade" $^{111}$In in 0.5 M acetate buffer solution, pH 6.0. Thin layer chromatography is used to separate the DTPA coupled antibody for calculation of the coupling efficiency. The DTPA-coupled antibodies can be stored at 4°C until needed for binding with metallic radionuclides such as $^{111}In+3$, $^{212}Bi+3$ and $^{68}Ga+3$, for example.

This invention is further illustrated by the following specific, but non-limiting examples. Temperatures are given in degrees Centigrade and percents as weight percents unless otherwise specified.

## EXAMPLE 1

*Polyclonal Anti-(fetal fibronectin) Antibody*

Fetal fibronectin is purified from amniotic fluid as described by Engvall and Ruoslahti, *Int.J.Cancer.* **20**:1-5 (1977).

The anti-(fetal fibronectin) antibodies are elicited in rabbits using the immunization techniques and schedules described in the literature, e.g., Stollar, *Meth.Enzym.* **70**:70 (1980), immunizing the rabbits with the fetal fibronectin antigen. The antiserum is screened in a solid phase assay similar to that used for monoclonal antibodies, e.g., as described by Lange et al, *Clin.Exp.Immunol.* **25**:191 (1976) and Pisetsky et al, *J.Immun.Meth.* **41**:187 (1981).

The IgG fraction of the antisera is purified further by affinity chromatography using CNBr-Sepharose Trade Mark 4B (Pharmacia Fine Chemicals) to which has been coupled fetal fibronectin. The method used for coupling is that recommended by the gel manufacturer, AFFINITY CHROMATOGRAPHY. Pharmacia Fine Chemicals, pp 15-18.

The column is equilibrated with from 2 to 3 volumes of buffer (0.01 M PBS, pH 7.2), and the anti-(fetal fibronectin) antibody containing solution is then applied to the column. The absorbency of the eluate is monitored at 280 nm until protein no longer passes from the column. The column is then washed with 0.1 M glycine buffer, pH 2.5, to desorb the immunoaffinity bound anti-(fetal fibronectin) antibody. Peak protein fractions are collected, pooled and dialyzed against 0.01 M PBS, pH 7.2, for 24-36 hr at 4°C with multiple buffer changes.

If a higher purity is desired, the affinity purified IgG can be passed through an adult plasma fibronectin bound affinity column by the procedure described above to remove any antibodies which would cross-react with adult plasma fibronectins.

## EXAMPLE 2

*Monoclonal Anti-(fetal fibronectin) Antibody*

Using the purified fetal fibronectin obtained by the procedure of Example 1, mouse monoclonal antibodies to the fetal fibronectin are obtained using standard procedures of Galfre and Milstein, *Meth.Enzym.* **73**:1 (1981) and Matsuura, H. and Hakomori, S. et al, *Proc.Natl.Acad.Sci.USA.* **82**:6517-6521 (1985), using fetal fibronectin as the antigen for immunizing the mice. The monoclonal antibodies are screened using a modification of the techniques described in the literature, e.g., Lange et al, Clin.Exp.Immunol. 25:191 (1976) and Pisetsky et al, *J.Immun.Meth.* **41**:187 (1981).

Mouse monoclonal antibody is purified from ascites fluid or from hybridoma culture supernatants using Protein-A coupled Sepharose-4B (Pharmacia Fine Chemicals) according to the procedure of Tijsson, PRACTICE AND THEORY OF ENZYME IMMUNOASSAYS. Elsevier Science Publishers, pp 105-107 (1985).

## EXAMPLE 3

*Antibody Coated Microtiter Plate*

Goat F(ab')$_2$ anti-(mouse IgG) antibody (Tago) is diluted to 10 μg/mL in 0.05 M carbonate buffer, pH 9.6. 100 μL is dispersed into each well of an IMMULON Trade Mark II microtiter plate (Dynatech). The plate is covered and incubated 4 hours at room temperature or 4°C overnight. The plate is washed 4 times with Wash Buffer (0.02 M Tris HCl, 0.015 M NaCl, 0.05% TWEEN Trade Mark -20). The plate is then blocked by dispensing into each well 200 μL of Blocking Solution (0.01 M PBS, 1% BSA, 0.02% NaN$_3$, pH 7.4). Anti-(hCG) antibodies prepared according to the procedures described in U.S. Patents 3,171,783, 3,234,096, 3,236,732, 3,309,275 4,116,776, 4,123,509, 4,234,561, 4,256,629, 4,268,435, 4,310,455, or 4,313,871 are is diluted 1/10,000 with 0.01 M PBS-1% BSA, pH 7.4. 100 μL of the solution is dispensed into each well of the blocked microtiter plate. The wells are incubated, covered, for 2 hours at room temperature or overnight at 4°C. The plate is then washed 4 times with Wash Buffer as described above, and is then ready for immunoassay of samples.

## EXAMPLE 4

*Polyclonal Antibody Coated Microtiter Plate*

Rabbit anti-(fetal fibronectin) prepared and further purified to remove adult fibronectin cross-reactivity as described in Example 1 is diluted to 10 μg/m$_L$ in 0.05 M carbonate buffer, pH 9.6. 100 μL is dispersed into each well of of an IMMULON Trade Mark II microtiter plate (Dynatech). The plate is covered and incubated 4 hr at room temperature or 4°C overnight. The plate is washed 4 times with Wash Buffer (0.02 M Tris HCl, 0.015 M NaCl, 0.05% TWEEN Trade Mark -20), filling and emptying the wells completely with each use. The plate is then blocked by dispersing into each well 200 μL of a blocking solution (0.01 M PBS, 1% BSA, 0.02% NaN$_3$, pH 7.4) and incubating for 1 hr at room temperature. The wells are then washed 4 times with Wash Buffer, as described above. The plate is now ready for immunoassay of samples.

## EXAMPLE 5

*Monoclonal Antibody Coated Microtiter Plate*

Goat F(ab')$_2$ anti-(mouse IgG) antibody (Tago) was diluted to 10 μg/mL in 0.05 M carbonate buffer, pH 9.6. 100 μL was dispersed into each well of an IMMULON Trade Mark II microtiter plate (Dynatech). The plate was covered and incubated 4 hr at room temperature or 4°C overnight. The plate was washed 4 times with Wash Buffer as described in Example 4. The plate was then blocked by dispensing into each well the Blocking Solution as described in Example 4. Mouse monoclonal anti-(fetal fibronectin) ascites prepared as in Example 2 was diluted 1/5000 with 0.01 M PBS-1% BSA, pH 7.4. 100 μL of the solution was dispensed into each well of the blocked microtiter plate. The wells were incubated, covered, for 2 hr at room temperature or overnight at 4°C. The plate was then washed 4 times with Wash Buffer as described above, and was then ready for immunoassay of samples.

## EXAMPLE 6

*Enzyme Labeled Anti-(hCG) Antibody*

Anti-(hCG) antibody is prepared in accordance with the procedures of U.S. Patents 3,171,783, 3,234,096, 3,236,732 or 3,309,275, and is conjugated with alkaline phosphatase following the one-step glutaraldehyde procedure of Avrameas, *Immunochem.* **6**:43 (1969).

## EXAMPLE 7

*Enzyme Labeled Anti-(fibronectin) Antibody*

Anti-(fibronectin) antibody prepared in accordance with the procedures of U.S. Patent 4,325,867 or derived from ATCC HB 91 (American Type Culture Collection, Rockville, MD) is conjugated with alkaline phosphatase following the one-step glutaraldehyde procedure of Avrameas, *Immunochem.* **6**:43 (1969).

## EXAMPLE 8

*Pregnancy Test*

A sample is removed from the vaginal cavity in the vicinity of the cervical canal or cervical os, and assayed to determine the presence of a nonrestricted pregnancy antigen. The presence of such a nonrestricted pregnancy antigen indicates that the patient is pregnant.

Positive and negative controls are included in the test. The positive control is a test sample of known pregnancy antigen concentration, appropriately diluted to fall within the assay range (20 ng/mL to 5 µg/mL for a monoclonal based assay). The negative control is sample diluent. The Assay Standard is a sample of known pregnancy antigen concentration, serially diluted in sample diluent to provide a standard curve, ranging from 20 ng to 5 µg/mL.

Sampling swabs containing the test sample collected in the vicinity of the cervical os are immersed in 0.75 mL of a Storage Solution consisting of 0.05 M Tris-HCl, pH 7.4; 0.15 M NaCl, 0.02% NaN₃, 1% BSA, 500 Kallikrein Units/mL of aprotinin, 1 mM phenylmethylsulfonyl fluoride (PMSF) and 5 mM EDTA. The swabs are removed from the Solution for the assay, and the solution is centrifuged at 13,000 rpm for 5 minutes to remove particulate matter. The supernatant contains the soluble pregnancy antigens which were in the swab.

A microtiter plate prepared as in Example 3 is used for the assay. 100 µL of each standard, sample, positive and negative control are placed in separate wells and incubated 2 hours at room temperature. The plate is washed 4 times with Wash Buffer, described above. 100 µL of alkaline phosphatase-conjugated goat anti-(hCG) prepared as in Example 6 is diluted 1/1000 in Conjugate Buffer (0.02 M Tris-HCl, pH 8, 0.3 M NaCl, 0.05% TWEEN Trade Mark 20, 5% BSA, 0.02% NaN₃). 100 µL is dispensed into each well and incubated for 2 hours at room temperature. The plate is washed 4 times as previously described. 4 mg/mL of *p*-nitrophenylphosphate (PNPP) is used as the substrate. This is diluted in 0.18 M 2-amino-2-methyl-1-propanol (AMP) buffer, pH 9.5 with 0.12 mM MgCl₂. 100 µL is dispensed into each well of the microtiter plate. After 5 minutes incubation at room temperature, the reaction rate in milli-OD/min is read at 405 nm on a V-MAX™ kinetic microtiter plate reader (Molecular Devices).

A standard curve is constructed by correlating increasing reaction rate with increasing pregnancy antigen concentration in the standards. Unknowns are calculated directly from the curve or by using a pre-set computer program (Molecular Devices). The presence of pregnancy antigen in the test sample indicates pregnancy.

## EXAMPLE 9

*Alternate Pregnancy Tests*

Repeating the procedure of Example 8 but replacing anti-(hCG) antibodies with anti-(SP1) antibodies produced as described in Japanese Patent Applications 59174762 and 59214767 provides a determination of pregnancy by assaying SPI pregnancy antigen.

Repeating the procedure of Example 8 but replacing anti-(hCG) antibodies with anti-(EPF) (early pregnancy factor) antibodies produced according to the methods of World Patent WO 8605498 provides a determination of pregnancy by assaying EPF.

Repeating the procedure of Example 8 but replacing anti-(hCG) antibodies with anti-(hPL) antibodies produced as described in U.S. Patent 3,892,841 provides a determination of pregnancy by assaying hPL.

Repeating the procedure of Example 8 but replacing anti-(hCG) antibodies with anti-(Protein B) antibodies produced as described in U.S. Patent 4,554,256 provides a determination of pregnancy by assaying Protein B.

Repeating the procedure of Example 8 but replacing anti-(hCG) antibodies with anti-(PP13) antibodies prepared as described in U.S. Patent 4,500,451 provides a determination of pregnancy by assaying PP13 pregnancy antigen.

## EXAMPLE 10

*Alternate Pregnancy Tests*

Repeating the procedure of Example 8 but replacing anti-(hCG) antibodies with anti-($\alpha$-fetoprotein) antibodies produced as described in Uotila, M. et al, *Mol.Immunol.* **17**:791 (1980) and Uotila, M. et al, *J.Immunol.Meth.* **42**:11 (1981) provides a determination of pregnancy by assaying $\alpha$-fetoprotein pregnancy antigen.

Repeating the procedure of Example 8 but replacing anti-(hCG) antibodies with anti-(MP1) antibodies produced as described in European Patent application 125514 (Nov. 21, 1984) provides a determination of pregnancy by assaying MPI pregnancy antigen.

Repeating the procedure of Example 8 but replacing anti-(hCG) antibodies with anti-(PP17) antibodies produced as described in U.S. Patent 4,468,345 provides a determination of pregnancy by assaying PP17 pregnancy antigen.

Repeating the procedure of Example 8 but replacing anti-(hCG) antibodies with anti-(PP20) antibodies produced as described in U.S. Patent 4,592,863 provides a determination of pregnancy by assaying PP20 pregnancy antigen.

Repeating the procedure of Example 8 but replacing anti-(hCG) antibodies with anti-(PLAP) (placental alkaline phosphatase) antibodies produced as described in Millan, J. et al, PLACENTAL PROTEINS (p. 432) provides a determination of pregnancy by assaying PLAP pregnancy antigen.

## EXAMPLE 11

*Pregnancy Test*

A sample is removed from the vaginal cavity in the vicinity of the cervical canal or cervical os, and assayed to determine the presence of a fetal restricted antigen. The presence of such a fetal restricted antigen in the sample indicates that the patient is pregnant.

Positive and negative controls were included in the test. The positive control was amniotic fluid of know fetal fibronectin concentration, appropriately diluted to fall within the assay range (20 ng/mL to 5 $\mu$g/mL for a monoclonal based assay). The negative control was sample diluent. The Assay Standard was amniotic fluid of known fibronectin concentration, serially diluted in sample diluent to provide a standard curve, ranging from 20 ng to 5 $\mu$g/mL.

The sample diluent consisted of 0.05 M Tris-HCl, pH 7.4; 0.15 M NaCl, 0.02% $NaN_3$, 1% BSA, 500 Kallikrein Units/mL of aprotinin, 1 mM phenylmethylsulfonyl fluoride (PMSF) and 5 mM EDTA.

Swab samples collected in the vicinity of the cervical os were immersed in 0.75 mL of sample diluent in a collection vial. The swabs were removed from the solution for the assay, and the solution was centrifuged at 13,000 rpm for 5 minutes to remove particulate. The supernatant contained any fetal fibronectin which was in the swab.

A microtiter plate prepared as in Example 5 is used for the assay. 100 $\mu$L of each standard, sample, positive and negative control were placed in separate wells and incubated 2 hours at room temperature. The plate was washed 4 times with Wash Buffer as described in Examples 4 and 5. 100$\mu$L of alkaline phosphatase-conjugated goat anti-(human fibronectin) prepared as in Example 7 was diluted 1/1000 in Conjugate Buffer (0.02 M Tris-HCl, pH 8, 0.3 M NaCl, 0.05% TWEEN Trade Mark 20, 5% BSA, 0.02% $NaN_3$).

100 $\mu$L was dispensed into each well and incubated for 2 hr at room temperature. The plate was washed 4 times as previously described. 4 mg/mL of *p*-nitrophenylphosphate (PNPP) was used as the substrate. This was diluted in 0.18 M 2-amino-2-methyl-1-propanol (AMP) buffer, pH 9.5 with 0.12 mM $MgCl_2$. 100 $\mu$L was dispensed into each well of the microtiter plate. After a 5 minute incubation at room temperature, the reaction rate in milli-OD/min was read at 405 nm on a V-MAX™ kinetic microtiter plate reader (Molecular Devices).

A standard curve was constructed by correlating increasing reaction rate with increasing fibronectin concentration in the standards. Unknowns were calculated directly from the curve or by using a pre-set computer program (Molecular Devices).

Samples obtained before week 20 of pregnancy which demonstrate significant fetal fibronectin in the test sample indicate normal uterine pregnancy. Samples in which significant amounts of fetal fibronectin are absent indicate that normal uterine pregnancy is not present.

## EXAMPLE 12

*Ectopic Pregnancy Test*

The procedure of Example 11 is repeated with samples collected in the vicinity of the cervical os of a patient who has tested positive for pregnancy, and who is suspected of having an ectopic pregnancy.

Samples obtained before week 20 of pregnancy which demonstrate a lack of significant fetal fibronectin in the test sample indicate the presence of ectopic pregnancy.

## EXAMPLE 13

*Product of Therapeutic Abortion Test*

Samples obtained during a therapeutic abortion are tested to verify that fetal materials have been removed from the uterus.

Positive and negative controls are included in the test. The positive control, amniotic fluid having a known concentration of fetal fibronectin is diluted 1/10, 1/30, 1/90, 1/270, 1/710 and 1/2130 in Storage Solution. The Storage Solution consisting of 0.05 M Tris-HCl, pH 7.4; 0.15 M NaCl, 0.02% $NaN_3$, 1% BSA, 500 Kallikrein Units/mL of aprotinin, 1 mM phenylmethylsulfonyl fluoride (PMSF) and 5 mM EDTA. This Storage Solution is described in U.S. Patent Application 244,969, filed September 15, 1988. The negative control is first trimester maternal plasma diluted 1/5 and 1/10 in Storage Solution. Storage Solution is used as the negative background control.

A sample of tissue or other material removed during a therapeutic abortion is dispersed in 0.75 mL of Storage Solution. The sample solutions are centrifuged in 1 mL microfuge tubes (13,000 rpm for 5 minutes) to remove particulate matter.

A microtiter plate prepared as in Example 5 is used for the assay. 100 μL of each standard, sample, positive and negative control are placed in separate wells and incubated 2 hours at room temperature. The plate is washed 4 times with Wash Buffer, described in Example 4. 100 μL of alkaline phosphatase-conjugated goat anti-(hCG) prepared as in Example 6 is diluted 1/1000 in Conjugate Buffer (0.02 M Tris-HCl, pH 8, 0.3 M NaCl, 0.05% TWEEN Trade Mark 20, 5% BSA, 0.02% $NaN_3$). 100 μL is dispensed into each well and incubated for 2 hours at room temperature. The plate is washed 4 times as previously described. 4 mg/mL of *p*-nitrophenylphosphate (PNPP) is used as the substrate. This is diluted in 0.18 M 2-amino-2-methyl-1-propanol (AMP) buffer, pH 9.5 with 0.12 mM $MgCl_2$. 100 μL is dispensed into each well of the microtiter plate. After 5 minutes incubation at room temperature, the reaction rate in milli-OD/min is read at 405 nm on a V-MAX™ kinetic microtiter plate reader (Molecular Devices Corp., Palo Alto, CA).

A standard curve is constructed by correlating increasing reaction rate with increasing fetal restricted antigen concentration in the standards. Unknowns are calculated directly from the curve or by using a pre-set computer program (Molecular Devices).

In samples containing products of conception, significant amounts of fetal fibronectin are found, confirming the existence of normal pregnancy and the termination thereof. If, in a pregnant patient, significant amounts of fetal fibronectin are absent, the possibility of an ectopic pregnancy is indicated.

## EXAMPLE 14

*Spontaneous Abortion Test*

The procedure of Example 13 is repeated with samples of vaginal discharge obtained during a suspected spontaneous abortion of a normal pregnancy. Samples from a spontaneous abortion (miscarriage) demonstrate significant levels of fetal fibronectin in the discharge. If fetal fibronectin is not detected in significant levels in the discharge, a continuing pregnancy is indicated.

## EXAMPLE 15

*Preterm Labor / Ruptured Membranes Sandwich Immunoassay*

The procedure of Example 13 is repeated with a test sample obtained during weeks 20-38 of pregnancy. The presence of significant amounts of fetal fibronectin in the sample indicates that the fetal membranes may have been breached, and that preterm labor is likely.

30

## Claims

1. A method for determining the presence and/or status of a pregnancy, comprising determining the presence of (a) a compound or material which is formed either by placental tissue or by maternal tissue in the uterus in reaction to pregnancy and which is either not normally present in plasma, serum or urine when a woman is not pregnant, or which is present in maternal plasma, serum or urine in increased amounts during pregnancy, (a "non-restricted pregnancy antigen"), or (b) a fetal or placental derived material which is either not present in maternal serum, plasma or urine or is not present in significant amounts in maternal serum, plasma or urine, (a "fetal restricted antigen"), in a sample, characterized in that the sample is derived from the vaginal cavity in the vicinity of the posterior fornix, cervical canal or cervical os.

2. A method for determining pregnancy during the first trimester comprising determining the presence of a compound or material which is formed either by placental tissue or by maternal tissue in the uterus in reaction to pregnancy, and which is either not normally present in plasma, serum or urine when a woman is not pregnant, or which is present in maternal plasma, serum or urine in increased amounts during pregnancy, (a "non-restricted pregnancy antigen"), in a sample, characterized in that the sample is derived from the vicinity of the cervical canal or cervical os.

3. A method for determining normal uterine pregnancy during the first 20 weeks of pregnancy comprising determining the presence of a fetal or placental derived material which is either not present in maternal serum, plasma or urine or is not present in significant amounts in maternal serum, plasma or urine, (a "fetal restricted antigen"), in a sample, characterized in that the sample is derived from the vicinity of the cervical canal or cervical os.

4. A method for determining fallopian pregnancy comprising determining the absence of a fetal or placental derived material which is either not present in maternal serum, plasma or urine or is not present in significant amounts in maternal serum, plasma or urine, (a "fetal restricted antigen"), in a sample, characterized in that the sample is derived from the vicinity of the cervical canal or cervical os of a pregnant patient during the first 20 weeks of pregnancy.

5. A method for determining ex vivo products of conception comprising determining the presence of a fetal or placental derived material which is either not present in maternal serum, plasma or urine or is not present in significant amounts in maternal serum, plasma or urine, (a "fetal restricted antigen"), in a sample, characterized in that the sample is expelled or removed from the uterus.

6. A method for determining increased risk of pre-term labour or fetal membrane rupture comprising determining the presence of a fetal or placental derived material which is either not present in maternal serum, plasma or urine or is not present in significant amounts in maternal serum, plasma or urine, (a "fetal restricted antigen"), in a sample, characterized in that the sample is derived from the vicinity of the posterior fornix, cervical canal or cervical os of a patient after week 20 of pregnancy.

7. The method of claim 1 or claim 2 comprising
   a) contacting the sample with an anti-(pregnancy antigen) antibody for a time sufficient to permit antigen-antibody binding to occur; and
   b) determining the presence of said binding.

8. The method of claim 1,2 or 7 comprising the steps of
   a) contacting the sample with an insoluble support to which anti-(pregnancy antigen) antibody is adhered for a time sufficient to permit antigen antibody binding to occur, and removing the sample from the support;
   b) contacting the insoluble support with an anti-(pregnancy antigen) antibody for a time sufficient to permit antigen-antibody binding to occur, and removing unbound anti-(pregnancy antigen) antibody from the support; and
   c) determining the presence of anti-(pregnancy antigen) antibody on the insoluble support.

9. The method of claim 1,3,4,5 or 6 comprising the steps of
   a) contacting the sample with an insoluble support to which anti-(fetal restricted antigen) antibody is adhered for a time sufficient to permit antigen-antibody binding to occur, and removing the sample from the support;

b) contacting the insoluble support with an anti-(fetal restricted antigen) antibody or anti-(fetal restricted antigen class) antibody for a time sufficient to permit antigen-antibody binding to occur, and removing unbound anti-(fetal restricted antigen) antibody from the support; and

c) determining the presence of anti-(fetal restricted antigen) antibody on the insoluble support.

10. The method of claim 1,3,4,5 or 6 comprising the steps of

a) contacting the sample with an insoluble support to which anti-(fetal restricted antigen class) antibody is adhered for a time sufficient to permit antigen-antibody binding to occur, and removing the sample from the support;

b) contacting the insoluble support with an anti-(fetal restricted antigen) antibody for a time sufficient to permit antigen-antibody binding to occur, and removing unbound anti-(fetal restricted antigen) antibody from the support; and

c) determining the presence of anti-(fetal restricted antigen) antibody on the insoluble support.

11. The method of claim 1,2,7 or 8 wherein the pregnancy antigen is selected from the group consisting of hCG, hCT, hPL, SP1, PAPP-A, PAPP-B, HSAP, PLAP, CAP, PP5, $PAMG_1$, $PAMG_2$, $\beta_1$-PAM, $\alpha_2$-PAM, hCLRF, $\alpha$-fetoprotein EPF, MP1, PP13, PP17, PP20, somatostatin, and mixtures thereof.

12. The method of claim 1,3,4,5,6,9 or 10 wherein the fetal restricted antigen determined in the sample is fetal fibronectin.

13. The method of claim 8,9 or 10 wherein the reagent antibody of step (b) has a physically detectable label.

14. A pregnancy antigen testing kit comprising combinations of a sampling device for collecting a test sample from the vicinity of the cervical canal or cervical os with (a) an anti-(pregnancy antigen) antibody adhered to an insoluble support; (b) a combination of anti-(pregnancy antigen) antibody adhered to an insoluble support, and either a labeled anti-(pregnancy antigen) antibody, a labeled anti-(pregnancy antigen class) antibody, or a labeled reagent pregnancy antigen; or (c) a reagent pregnancy antigen adhered to an insoluble support and a labeled anti-(pregnancy antigen) antibody; a "pregnancy antigen" being a compound or material which is formed either by placental tissue or by maternal tissue in the uterus in reaction to pregnancy and which is either not normally present in plasma, serum or urine when a woman is not pregnant, or which is present in material plasma, serum or urine in increased amounts during pregnancy.

15. A kit for determining a fetal restricted antigen in a test sample, comprising combinations of a sampling device for collecting a test sample from the vicinity of the cervical canal and cervical os and (a) an anti-(fetal restricted antigen) antibody, and anti-(fetal restricted antigen class) antibody, or reagent fetal restricted antigen adhered to an insoluble support; (b) a combination of anti-(fetal restricted antigen) antibody adhered to an insoluble support, and either a labeled anti-(fetal restricted antigen) antibody, a labeled anti-(fetal restricted antigen class) antibody, or a labeled reagent fetal restricted antigen; (c) a combination of anti-(fetal restricted antigen class) antibody adhered to an insoluble support with a labelled anti-(fetal restricted antigen) antibody; or (d) a reagent fetal restricted antigen adhered to an insoluble support and a labelled anti-(fetal restricted antigen) antibody; a "fetal restricted antigen" being a fetal or placental derived material which is either not present in maternal serum, plasma or urine or is not present in significant amounts in maternal serum, plasma or urine.

16. A kit of claim 14 or 15 further including a labelled anti-(sandwiching antibody) antibody.

17. A kit comprising an anti-(fetal restricted antigen) antibody and a sampling device for collecting a test sample from the vicinity of the cervical canal or cervical os, a "fetal restricted antigen" being a fetal or placental derived material which is either not present maternal serum, plasma or urine or is not present in significant amounts in maternal serum, plasma or urine.

## Patentansprüche

1. Verfahren zur Bestimmung des Vorliegens von und/oder des Stadiums einer Schwangerschaft, umfassend das Nachweisen der Gegenwart (a) einer Verbindung oder eines Stoffes, die/der entweder von Plazentagewebe oder von Gewebe im Uterus der Mutter als Reaktion auf Schwangerschaft gebildet wird und die/der entweder normalerweise nicht in Plasma, Serum oder Harn vorkommt, wenn eine Frau nicht

EP 0 316 919 B1

schwanger ist, oder die/der während der Schwangerschaft in erhöhten Mengen im Plasma, Serum oder Harn der Mutter vorhanden ist, (eines "nicht beschränkten Schwangerschafts-Antigens"), oder (b) eines von Fötus oder Plazenta stammenden Stoffs, der entweder nicht oder nicht in signifikanten Mengen im Serum, Plasma oder Harn der Mutter vorkommt, (eines "fötal beschränkten Antigens"), in einer Probe, dadurch gekennzeichnet, daß die Probe aus dem Vaginalraum in der Nähe des hinteren Scheidengewölbes, dem Cervikalkanal oder dem Muttermund, stammt.

2. Verfahren zum Nachweis von Schwangerschaft im ersten Trimenon, umfassend das Nachweisen der Gegenwart (a) einer Verbindung oder eines Stoffes, die/der entweder von Plazentagewebe oder von Gewebe im Uterus der Mutter als Reaktion auf Schwangerschaft gebildet wird und die/der entweder normalerweise nicht in Plasma, Serum oder Harn vorkommt, wenn eine Frau nicht schwanger ist, oder die/der während der Schwangerschaft in erhöhten Mengen im Plasma, Serum oder Harn der Mutter vorhanden ist, (eines "nicht beschränkten Schwangerschafts-Antigens"), in einer Probe, dadurch gekennzeichnet, daß die Probe aus der Nähe des Cervikalkanals oder Muttermunds stammt.

3. Verfahren zum Nachweis von normaler Intrauteringravidität in den ersten 20 Wochen der Schwangerschaft, umfassend das Nachweisen des Gegenwart eines vom Fötus oder der Plazenta stammenden Stoffs, der entweder nicht oder nicht in signifikanten Mengen im Serum, Plasma oder Harn der Mutter vorkommt, (eines "fötal beschränkten Antigens"), in einer Probe, dadurch gekennzeichnet, daß die Probe aus der Nähe des Cervikalkanals oder des Muttermunds stammt.

4. Verfahren zum Nachweis von Eileiterschwangerschaft, umfassend das Nachweisen der Abwesenheit eines vom Fötus oder der Plazenta stammenden Stoffs, der entweder nicht oder nicht in signifikanten Mengen im Serum, Plasma oder Harn der Mutter vorkommt, (eines "fötal beschränkten Antigens"), in einer Probe, dadurch gekennzeichnet, daß die Probe aus der Nähe des Cervikalkanals oder des Muttermunds einer schwangeren Patientin während der ersten 20 Wochen der Schwangerschaft stammt.

5. Verfahren zum Nachweis von ex vivo-Empfängnisprodukten, umfassend das Nachweisen der Gegenwart eines vom Fötus oder der Plazenta stammenden Stoffs, der entweder nicht oder nicht in signifikanten Mengen im Serum, Plasma oder Harn der Mutter vorkommt, (eines "fötal beschränkten Antigens"), in einer Probe, dadurch gekennzeichnet, daß die Probe vom Uterus abgegeben oder daraus entnommen wird.

6. Verfahren zum Nachweis erhöhten Risikos von vorzeitigen Wehen oder Fruchtblasensprung, umfassend das Nachweisen der Gegenwart eines vom Fötus oder der Plazenta stammenden Stoffs, der entweder nicht oder nicht in signifikanten Mengen im Serum, Plasma oder Harn der Mutter vorkommt, (eines "fötal beschränkten Antigens"), in einer Probe, dadurch gekennzeichnet, daß die Probe aus der Nähe des hinteren Scheidengewölbes, dem Cervikalkanal oder dem Muttermund einer Patientin während der ersten 20 Wochen der Schwangerschaft stammt.

7. Verfahren nach Anspruch 1 oder 2, umfassend
   (a) das In-Kontakt-Bringen der Probe mit einem Anti-(Schwangerschafts-Antigen)-Antikörper über eine ausreichende Zeitspanne, um die Ausbildung der Antigen-Antikörper-Bindung zu ermöglichen; und
   (b) das Nachweisen der Gegenwart dieser Bindung.

8. Verfahren nach Anspruch 1, 2 oder 7, umfassend die Schritte:
   (a) das In-Kontakt-Bringen der Probe mit einem unlöslichen Träger, an dem Anti-(Schwangerschafts-Antigen)-Antikörper angebracht wurde, über eine ausreichende Zeitspanne, um die Ausbildung der Antigen-Antikörper-Bindung zu ermöglichen, und das Entfernen der Probe vom Träger;
   (b) das In-Kontakt-Bringen des unlöslichen Trägers mit einem Anti-(Schwangerschafts-Antigen)-Antikörper über eine ausreichende Zeitspanne, um die Ausbildung der Antigen-Antikörper-Bindung zu ermöglichen, und das Entfernen von ungebundenem Anti-(Schwangerschafts-Antigen)-Antikörper vom Träger; und
   (c) das Nachweisen der Gegenwart von Anti-(Schwangerschafts-Antigen)-Antikörper auf dem unlöslichen Träger.

9. Verfahren nach Anspruch 1, 3, 4, 5 oder 6, umfassend die Schritte:
   (a) das In-Kontakt-Bringen der Probe mit einem unlöslichen Träger, an dem Antikörper gegen fötal beschränktes Antigen angebracht wurde, über eine ausreichende Zeitspanne, um die Ausbildung der Antigen-Antikörper-Bindung zu ermöglichen, und das Entfernen der Probe vom Träger;

33

(b) das In-Kontakt-Bringen des unlöslichen Trägers mit einem Antikörper gegen fötal beschränktes Antigen oder gegen die Klasse fötal beschränkter Antigene über eine ausreichende Zeitspanne, um die Ausbildung der Antigen-Antikörper-Bindung zu ermöglichen, und das Entfernen von ungebundenem Antikörper gegen fötal beschränktes Antigen vom Träger; und

(c) das Nachweisen der Gegenwart von Antikörper gegen fötal beschränktes Antigen auf dem unlöslichen Träger.

10. Verfahren nach Anspruch 1, 3, 4, 5 oder 6, umfassend die Schritte:

(a) das In-Kontakt-Bringen der Probe mit einem unlöslichen Träger, an dem Antikörper gegen die Klasse fötal beschränkter Antigene angebracht wurde, über eine ausreichende Zeitspanne, um die Ausbildung der Antigen-Antikörper-Bindung zu ermöglichen, und das Entfernen der Probe vom Träger;

(b) das In-Kontakt-Bringen des unlöslichen Trägers mit einem Antikörper gegen fötal beschränktes Antigen über eine ausreichende Zeitspanne, um die Ausbildung der Antigen-Antikörper-Bindung zu ermöglichen, und das Entfernen von ungebundenem Antikörper gegen fötal beschränktes Antigen vom Träger; und

(c) das Nachweisen der Gegenwart von Antikörper gegen fötal beschränktes Antigen auf dem unlöslichen Träger.

11. Verfahren nach Anspruch 1, 2, 7 oder 8, worin das Schwangerschafts-Antigen aus der aus hCG, hCT, hPL, SP1, PAPP-A, PAPP-B, HSAP, PLAP, CAP, PP5, $PAMG_1$, $PAMG_2$, $\beta_1$-PAM, $\alpha_2$-PAM, hCLRF, $\alpha$-Fetoprotein, EPF, MP1, PP13, PP17, PP20, Somatostatin und Gemischen davon bestehenden Gruppe ausgewählt wird.

12. Verfahren nach Anspruch 1, 3, 4, 5, 6, 9 oder 10, worin das in der Probe nachgewiesene fötal beschränkte Antigen fötales Fibronectin ist.

13. Verfahren nach Anspruch 8, 9 oder 10, worin der reagierende Antikörper aus Schritt (b) eine physikalisch nachweisbare Markierung aufweist.

14. Schwangerschafts-Antigen-Test-Set, umfassend Kombinationen einer Probennahme-Vorrichtung zum Ziehen einer Testprobe aus der Nähe des Cervikalkanals oder des Muttermunds mit (a) einem an einem unlöslichen Träger angebrachten Anti-(Schwangerschafts-Antigen)-Antikörper; (b) einer Kombination von einem an einem unlöslichen Träger angebrachten Anti-(Schwangerschafts-Antigen)-Antikörper und entweder einem markierten Anti-(Schwangerschafts-Antigen)-Antikörper, einem markierten Antikörper gegen die Klasse der Schwangerschafts-Antigene oder einem markierten reagierenden Schwangerschafts-Antigen; oder (c) einem an einem unlöslichen Träger angebrachten reagierenden Schwangerschafts-Antigen und einem markierten Anti-(Schwangerschafts-Antigen)-Antikörper; wobei ein "Schwangerschafts-Antigen" eine Verbindung oder ein Stoff ist, die/der entweder von Plazentagewebe oder von Gewebe im Uterus der Mutter als Reaktion auf Schwangerschaft gebildet wird und die/der entweder normalerweise nicht in Plasma, Serum oder Harn vorkommt, wenn eine Frau nicht schwanger ist, oder die/der während der Schwangerschaft in erhöhten Mengen im Plasma, Serum oder Harn der Mutter vorhanden ist.

15. Set zum Nachweis eines fötal beschränkten Antigens in einer Testprobe, umfassend Kombinationen einer Probennahme-Vorrichtung zum Ziehen einer Testprobe aus der Nähe des Cervikalkanals oder des Muttermunds und (a) einen Antikörper gegen fötal beschränktes Antigen und einen Antikörper gegen die Klasse fötal beschränkter Antigene oder ein reagierendes fötal beschränktes Antigen, an einem unlöslichen Träger angebracht; (b) eine Kombination von einem an einem unlöslichen Träger angebrachten Antikörper gegen fötal beschränktes Antigen und entweder einem markierten Antikörper gegen fötal beschränktes Antigen, einem markierten Antikörper gegen die Klasse fötal beschränkter Antigene oder einem markierten reagierenden fötal beschränkten Antigen; (c) eine Kombination von an einem unlöslichen Träger angebrachtem Antikörper gegen die Klasse fötal beschränkter Antigene und einem markierten Antikörper gegen fötal beschränktes Antigen; oder (d) ein an einem unlöslichen Träger angebrachtes reagierendes fötal beschränktes Antigen und ein markierter Antikörper gegen fötal beschränktes Antigen; wobei ein "fötal beschränktes Antigen" ein entweder vom Fötus oder der Plazenta stammender Stoff ist, der entweder nicht oder nicht in signifikanten Mengen im Serum, Plasma oder Harn der Mutter vorkommt.

16. Set nach Anspruch 14 oder 15, das weiters einen Anti-(Sandwiching-Antikörper)-Antikörper umfaßt.

17. Set, umfassend einen Antikörper gegen fötal beschränktes Antigen und eine Probennahme-Vorrichtung zum Ziehen einer Testprobe aus der Nähe des Cervikalkanals oder des Muttermunds, wobei ein fötal beschränktes Antigen" ein entweder vom Fötus oder der Plazenta stammender Stoff ist, der entweder nicht oder nicht in signifikanten Mengen im Serum, Plasma oder Harn der Mutter vorkommt.

**Revendications**

1. Méthode pour déterminer la présence/ou l'état d'une grossesse, consistant à déterminer la présence de (a) un composant ou matériau qui est formé soit par le tissu placentaire ou par le tissu maternel dans l'utérus en réaction à la grossesse et qui n'est pas normalement présent dans le plasma, le sérum ou les urines quand une femme n'est pas enceinte ou qui est présent dans le plasma, le sérum ou les urines maternels en quantités accrues pendant la grossesse (un "antigène de grossesse non restreint") ou (b) un matériau foetal ou placentaire qui est soit non présent dans le sérum, le plasma ou les urines maternels ou qui n'est pas présent en quantités importantes dans le sérum, le plasma ou les urines maternels (un "antigène foetal restreint") dans un échantillon, caractérisée en ce que l'échantillon est dérivé de la cavité vaginale à proximité du fornix postérieur, du canal cervical ou de l'orifice cervical.

2. Méthode pour déterminer la grossesse pendant le premier trimestre, consistant à déterminer la présence d'un composé ou d'un matériau qui est formé soit par le tissu placentaire ou par le tissu maternel dans l'utérus en réaction à la grossesse, et qui n'est soit pas normalement présent dans le plasma, le sérum ou les urines quand une femme n'est pas enceinte ou bien qui est présent dans le plasma, le sérum ou les urines maternels en quantités accrues pendant la grossesse (un "antigène de grossesse non restreint") dans un échantillon, caractérisée en ce que l'échantillon est dérivé de la proximité du canal cervical ou de l'orifice cervical.

3. Méthode pour déterminer une grossesse utérine normale pendant les 20 premières semaines de grossesse, consistant à déterminer la présence d'un matériau dérivé du foetus ou du placenta qui est soit non présent dans le sérum, le plasma ou les urines maternels ou bien qui n'est pas présent en quantités importantes dans le sérum, le plasma ou les urines maternels (un "antigène foetal restreint") dans un échantillon, caractérisée en ce que l'échantillon est dérivé de la proximité du canal cervical ou de l'orifice cervical.

4. Méthode pour déterminer une grossesse extra-utérine consistant à déterminer l'absence d'un matériau dérivé du foetus ou du placenta qui est soit non présent dans le sérum, le plasma ou les urines maternels ou bien qui n'est pas présent en quantités importantes dans le sérum, le plasma ou dans les urines maternels (un "antigène foetal restreint") dans un échantillon, caractérisée en ce que l'échantillon est dérivé de la proximité du canal cervical ou de l'orifice cervical d'une patiente enceinte pendant les 20 premières semaines de la grossesse.

5. Méthode pour déterminer <u>ex vivo</u> des produits de conception, consistant à déterminer la présence d'un matériau dérivé du foetus ou du placenta qui est soit non présent dans le sérum, le plasma ou les urines maternels ou bien qui est non présent en quantités importantes dans le sérum, le plasma ou les urines maternels (un "antigène foetal restreint") dans un échantillon, caractérisée en ce que l'échantillon est expulsé ou retiré de l'utérus.

6. Méthode pour déterminer un risque accru de travail avant terme ou de rupture de la membrane foetale, consistant à déterminer la présence d'un matériau dérivé du foetus ou du placenta qui est soit non présent dans le sérum, le plasma ou les urines maternels ou qui n'est pas présent en quantités importantes dans le sérum, le plasma ou les urines maternels (un "antigène foetal restreint") dans un échantillon, caractérisée en ce que l'échantillon est dérivé de la proximité du fornix postérieur, du canal cervical ou de l'orifice cervical d'une patiente au bout de la 20ème semaine de grossesse.

7. Méthode de la revendication 1 ou de la revendication 2 consistant à
a) mettre l'échantillon en contact avec un anticorps anti(antigène de la grossesse) pendant un temps suffisant pour 5 permettre à la liaison antigène-anticorps de se produire ; et
b) déterminer la présence de ladite liaison.

8. Méthode de la revendication 1, 2 ou 7 comprenant les étapes de :

a) mettre l'échantillon en contact avec un support insoluble auquel adhère un anticorps anti-(antigène de la grossesse) pendant un temps suffisant pour permettre à la liaison antigène-anticorps de se produire et retirer l'échantillon du support ;

b) mettre le support insoluble en contact avec un anticorps anti-(antigène de la grossesse) pendant un temps suffisant pour permettre à la liaison antigène-anticorps de se produire et retirer l'anticorps anti-(antigène) de la grossesse non lié du support ; et

c) déterminer la présence d'un anticorps anti-(antigène) de la grossesse sur le support insoluble.

9. Méthode de la revendication 1, 3, 4, 5 ou 6 comprenant les étapes de

a) mettre l'échantillon en contact avec un support insoluble auquel adhère un anticorps anti (antigène foetal restreint) pendant un temps suffisant pour permettre à la liaison antigène-anticorps de se produire et retirer l'échantillon du support ;

b) mettre le support insoluble en contact avec un anticorps anti-(antigène foetal restreint) ou un anticorps anti-(classe d'antigène foetal restreint) pendant un temps suffisant pour permettre à la liaison antigène-anticorps de se produire et retirer l'anticorps anti-(antigène foetal restreint) non lié du support et

c) déterminer la présence de l'anticorps anti-(antigène foetal restreint) sur le support insoluble.

10. Méthode de la revendication 1, 3, 4, 5 ou 6 comprenant les étapes de

a) mettre l'échantillon en contact avec un support insoluble auquel adhère un anticorps anti-(classe antigène foetal restreint) pendant un temps suffisant pour permettre à la liaison antigène-anticorps de se produire et retirer l'échantillon du support

b) mettre le support insoluble en contact avec un anticorps anti-(antigène foetal restreint) pendant un temps suffisant pour permettre à la liaison antigène-anticorps de se produire et retirer l'anticorps anti-(antigène foetal restreint) non lié du support et

c) déterminer la présence de l'anticorps anti-(antigène foetal restreint) sur le support insoluble.

11. Méthode la revendication 1, 2, 7 ou 8 où l'antigène de la grossesse est sélectionné dans le groupe consistant en hCG, hCT, hPL, SP1, PAPP-A, PAPP-B, HSAP, PLAP, CAP, PP5, $PAMG_1$, $PAMG_2$, $\beta_1$-PAM, $\alpha_2$-PAM, hCLRF, $\alpha$-foetoprotéine, EPF, MP1, PP13, PP17, PP20, somastostatine et leurs mélanges.

12. Méthode de la revendication 1, 3, 4, 5, 6, 9 ou 10 où l'antigène foetal restreint déterminé dans l'échantillon est la fibronectine foetale.

13. Méthode de la revendication 8, 9 ou 10 où l'anticorps réactif de l'étape (b) a un marqueur physiquement détectable.

14. Nécessaire de test de l'antigène de la grossesse comprenant des associations d'un dispositif d'échantillonnage pour collecter un échantillon de test de la proximité du canal cervical ou de l'orifice cervical avec a) un anticorps anti(antigène de la grossesse) adhérant à un support insoluble ; b) une association d'un anticorps anti-(antigène de la grossesse) adhérant à un support insoluble et soit un anticorps anti(antigène de la grossesse) marqué, un anticorps anti-classe d'antigène de la grossesse) marqué ou un antigène réactif marqué de la grossesse ; ou bien c) un antigène réactif de la grossesse adhérant à un support insoluble et un anticorps anti-(antigène de la grossesse) marqué ; un "antigène de la grossesse" étant un composé ou un matériau qui est formé soit par le tissu placentaire ou par le tissu maternel dans l'utérus, en réaction à la grossesse et qui n'est soit pas normalement présent dans le plasma, le sérum ou les urines lorsqu'une femme n'est pas enceinte ou qui est présent dans le plasma, le sérum ou les urines maternels en quantités accrues pendant la grossesse.

15. Nécessaire pour déterminer un antigène foetal restreint dans un échantillon de test, comprenant des associations d'un dispositif d'échantillonnage pour collecter un échantillon de test de la proximité du canal cervical ou de l'orifice cervical et a) un anticorps anti-(antigène foetal restreint) et un anticorps anti-(classe d'antigène foetal restreint) où un antigène réactif restreint adhérant à un support insoluble ; b) une association d'un anticorps anti-(antigène foetal restreint) adhérant à un support insoluble et soit un anticorps anti(antigène foetal restreint) marqué, un anticorps anti-(classe d'antigène foetal restreint) marqué ou un antigène foetal restreint réactif marqué ; c) une association d'un anticorps anti-(classe d'antigène foetal restreint) adhérant à un support insoluble avec un anticorps anti-(antigène foetal restreint) marqué ; ou d) un antigène foetal restreint réactif adhérant à un support insoluble et un antigène anti-(antigène foetal restreint) marqué ; un antigène foetal restreint étant un matériau dérivé du foetus ou du placenta qui est soit non présent dans le sérum, le plasma ou les urines maternels ou bien qui n'est pas présent en quantités importantes dans le sérum, le plasma ou les urines maternels.

16. Nécessaire de la revendication 14 ou 15 comprenant de plus un anti-anticorps (anticorps en sandwich) marqué.

17. Nécessaire comprenant un anticorps anti(antigène foetal restreint) et un dispositif d'échantillonnage pour recueillir un échantillon de test de la proximité du canal cervical ou de l'orifice cervical, un "antigène foetal restreint" étant un matériau dérivé du foetus ou du placenta qui est soit non présent dans le sérum, le plasma ou les urines maternels ou bien qui n'est pas présent en quantités importantes dans le sérum, le plasma ou les urines maternels.